# EUROPEAN PATENT SPECIFICATION

(11) **EP 1 694 862 B1**
(45) Date of publication and mention of the grant of the patent: **21.07.2010**
(21) Application number: 04806369.7
(22) Date of filing: 16.12.2004
(51) Int. Cl.: C12Q 1/68

(54) **METHODS AND COMPOSITIONS FOR ASSAYING MUTATIONS IN NUCLEIC ACIDS AND THEIR USES IN DIAGNOSIS OF GENETIC DISEASES AND CANCERS**
VERFAHREN UND ZUSAMMENSETZUNGEN ZUR BESTIMMUNG VON MUTATIONEN IN NUKLEINSÄURE UND DEREN VERWENDUNG IN DIAGNOSE VON GENETISCHEN KRANKHEITEN UND KREBS
METHODES ET COMPOSITIONS D'ESSAIS BIOLOGIQUES POUR DETECTER DES ACIDES NUCLEIQUES ET LEUR UTILISATIONS POUR LE DIAGNOSTIC DE MALADIES GENETIQUES ET DE CANCERS

(30) Priority: 16.12.2003 WO PCT/IB03/06016
(43) Date of publication of application: 30.08.2006
(73) Proprietor: Institut Curie, 75248 Paris Cedex 05 (FR); CENTRE NATIONAL DE LA RECHERCHE SCIENTIFIQUE, 75794 Paris Cedex (FR)
(72) Inventor: VIOVY, Jean-Louis, F-75013 Paris (FR)
(74) Representative: Le Coupanec, Pascale A.M.P.
(86) International application number: PCT/IB2004/004171
(87) International publication number: WO 2005/059175

(56) References cited:
- WO-A-99/10369
- WO-A-02/057492
- US-A- 4 794 075
- US-A- 5 876 941
- PULYAEVA HELENA ET AL: "Detection of a single base mismatch in double-stranded DNA by electrophoresis on uncrosslinked polyacrylamide gel" ELECTROPHORESIS, vol. 15, no. 8-9, 1994, pages 1095-1100, XP009034541 ISSN: 0173-0835
- KUTYAVIN I V ET AL: "3'-minor groove binder-DNA probes increase sequence specificity at PCR extension temperatures." NUCLEIC ACIDS RESEARCH. 15 JAN 2000, vol. 28, no. 2, 15 January 2000 (2000-01-15), pages 655-661, XP002318952 ISSN: 1362-4962
- [Online] FRED RUSSEL KRAMER: "Introduction to Molecular beacons" Database accession no. HTTP://WWW.MOLECULAR-BEACONS.ORG/INTRODUCT ION.HTML
- LEBOWITZ ET AL: "Carbodiimide modification of superhelical PM2 DNA: considerations regarding reaction at unpaired bases and unwinding of superhelicl DNA with chemical probes" NUCLEIC ACIDS RESEARCH, vol. 4, no. 6, 1977, pages 1695-1711,

## Description

The present invention concerns improved methods for assaying mutation(s) in nucleic acids, in particular point mutation in nucleic acid in duplex form.

Recent developments in genomics have raised considerable hopes for improvements in human health and biotechnology.

In medicine, for instance, the understanding and diagnosis of genetic diseases and cancers, or the study of infectious organisms, rely more and more on analysis of DNA and nucleic acids.

Biotechnology is also more and more dependent upon molecular genetic and nucleic acids high throughput analysis. In particular, mapping of genetic differences between individuals is of growing importance for forensic investigations, medical applications, biotechnology and food industry.

For example, detecting mutations leading to abnormal proteins can be essential for identifying the genetic origin of a disease. A number of inherited pathological conditions may be diagnosed before onset of symptoms, using methods for structural analyses of DNA. In cancer research, for example, the search of mutations in BRCA1 and BRCA2 genes, recognized to lead to strong increase in breast cancer, is now performed on a large scale. The gene APC is also known to lead to strong predisposition to colorectal cancer. Genetic screening can also be performed at an early age, or even *in utero,* for numerous heritable diseases. At present, 700 genetic diseases have been identified among which thalassemia or myopathy.

The identification and detailed analysis of acquired genetic disorders, such as arising in particular in cancer, is also raising the hopes of more efficient and personalized treatments, by means of "genetic mapping" of tumors. Large scale genetic screening of mutations and genetic variability, also called "genotyping", is also of paramount importance for detemining correlations between diseases and genes, in order to find new targets for therapy, in a pharmacogenomic approach.

Genetic screening can also be used for detecting pathogens, including identification of specific pathogenic varieties in medicine, food industry, veterinary or bioterrorism applications. For example, in food industry, the detection of genetically modified organism "GMO" in starting material or foodstuff is an increasing concern.

A need exists, therefore for a methodology to detect mutations in a DNA fragment relative to the wild type in an accurate, reproducible and reliable manner.

DNA molecules are linear polymers of subunits called nucleotides. Each nucleotide comprises a common cyclic sugar molecule, which is linked by phosphate group to the sugar of the adjoining nucleotide, and one of the different cyclic substituants called bases. The combination of the phosphate and base is called a nucleoside. The four bases commonly found in DNAs from natural sources are adenine, guanine, cytosine and thymidine, hereinafter referred to as A, G, C and T, respectively. The linear sequence of these bases in the DNA of an individual is its "genome". It involves coding regions, which bear the information for synthesis of proteins, regions for regulation of gene expression, and so called "non-coding" regions, the role of which being not fully understood.

In double-stranded DNA, the form adopted by DNA in the chromosomes of all cellular organisms, the two DNA strands are entwined in a precise helical configuration with the bases oriented inward, allowing interactions between bases from opposing strands. The two strands are held together in precise alignment mainly by hydrogen bonds which are permitted between bases by a complementarity of structures of specific pairs of bases. This structural complementarity is determined by the chemical natures and locations of substituents on each of the bases, leading in particular to a definite number and orientation of hydrogen bonds. Thus, in double-stranded DNA, normally each A on one strand has an attractive interaction with a T from the opposing strand, involving two hydrogen bonds, and each G has an attractive interaction with an opposing C involving three hydrogen bonds. In principle, they insure that DNA molecules are replicated and precise copies are passed on to the cell descendants during cell reproduction (mitosis), or to the offspring of the individual, when replication concerns the gametes (meiosis).

Occasionally, an incorrect base pairing does occur during replication, which, after further replication of the new strand, results in a double-stranded DNA offspring with a sequence containing a heritable single base difference from that of the parent DNA molecule. Such heritable changes are called genetic mutations, or more particularly in the present case, "point mutations". Mapping of genetic mutations involves both the detection of sequence differences between DNA molecules comprising substantially identical (i.e., homologous) base sequences, and also the physical localization of those differences within some subset of the sequences in the molecules being compared. Variations in the DNA sequence may also affect non-coding regions. In particular, highly variable such as short tandem repeats (STR) or Single nucleotide polymorphism (SNP), exist in non-coding regions, and are very useful in genotyping.

Detecting point mutations, and in particular substitutions, is particularly challenging, because they are very localized, and in many occurrences their position cannot be known in advance. Actually, for many mutations associated with diseases, the exact location of the mutation is not known a priori, and the whole coding sequence of the gene (generally representing several thousands or tens of thousands of bases) must be screened completely.

The most prominent technologies at present for nucleic analysis are capillary electrophoresis and hybridization arrays, also called "DNA or RNA chips". The two techniques are rather complementary: Hybridization methods are well adapted to ultra-high throughput and semi-quantitative evaluations on small sequences, whereas electrophoresis remains unchallenged for high resolution, for reproducibility and for the analysis of large molecules or fragments. Other systems, called "laboratories-on-chips" or "microfluidic systems", are also under development, and bear the promise of simpler, more cost-effective and more high-throughput analyses in the analysis of nucleic acids.

If the position of the mutation is known, one mostly uses single nucleotide primer extension, hybridization arrays, or quantitative PCR.

In single nucleotide primer extension, a sequencing reaction is performed, in conditions in which extension from the primer can start only if a given base (A,T,G, or C) follows the primer. The same reaction is performed for each base, and detection of extension is performed by a conventional sequencing electrophoresis.

In hybridization arrays, different "probe" DNA fragments or oligonucleotides encompassing the point mutation, and bearing all expected mutations, are arranged in a array on the surface of a "chip", and put in presence of the target nucleic acid to test (DNA or RNA). With suitable hybridization conditions, it is expected that the target will hybridize only with the probe bearing the exactly complementary sequence, thus identifying the sequence of the target. The main difficulty with this approach, is that the hybridization energy difference between the mutated and the normal (wild type) probe can be rather low, especially in the case of substitutions. It is thus difficult to find optimal conditions to get "yes or no" answers, and the difference in signal can be rather faint. This difficulty, combined with the relatively low reproducibility of hybridization arrays, leads to a significant error rate. In addition, the hybridization free energy depends rather strongly on the sequence. It is for instance, higher for sequences with a high GC content. Thus, a duplex with a high GC content and a mismatch, may have an affinity actually higher than a perfect duplex with the same length, but a low GC content. It is thus very difficult, if not impossible, to achieve a unique set of hybridization conditions (temperature and buffer) for testing in parallel many different sequences in a single array.

Various approaches have been proposed to improve the selectivity between matched and mismatched pairs in hybridization arrays. In Maskos et al., Nucleic Acids Research, 21, 4663-4669, 1993, for instance, the effort was applied to the choice of stringency of the buffer, but only with limited success. In Chiari et al., (HPCE 2003, San Diego, CA, Jan 17-22, 2003) a hybridization method is proposed, in which DNA probes are replaced by peptide nucleic acids (PNA). PNA are nucleic acids homologs, presenting a peptide (instead of phosphate) backbone. The hybridization energy per base pair is higher in a PNA/DNA pair than in an equivalent DNA/DNA pair, because the PNA backbone is neutral, so that electric repulsion is suppressed. It also appears that the use of PNA allows the use of shorter oligonucleotides, thus increasing the association energy difference between matched and mismatched pair, in the case of point mutations. PNAs, however, are very expensive, and the main strategies for constructing high density arrays are not transposable to the PNA chemistry. In WO 00/56916, Mogens et al. describe the use of another class of DNA analogs, locked nucleic acids (LNA). LNAs are nucleic acids in which the backbone is bi-cyclic. LNAs have a stronger affinity to complementary DNA than natural DNA, and also exhibit higher selectivity versus mismatches. However, the difficulties raised by PNA, such as cost and difficulties to make high density arrays are also present for LNA.

In fact, primer extension or hybridisation methods appear to be not well adapted to the search of mutations which position on the genome is not known.

Accordingly, prior and important efforts have been exerted in order to find efficient, fast and inexpensive methods for the detection of point mutation by capillary electrophoresis (for review, see e.g. Righetti, P.G., Gelfi, C., in " Analysis of Nucleic Acids by Capillary Electrophoresis, Heller, C, Ed., Chromatographia CE series, Vol 1Vieweg press, 1997, 255-271). The major techniques presently available are termed "Direct sequencing" and "Single Strand Conformation Polymorphism (SSCP)".

In the approach "Direct sequencing" the whole gene is sequenced totally for each patient, a powerful but costly method. In addition, due to the presence of heterozygoty, mutated patient sequences are not « pure », and the interpretation of data is not as straightforward as for conventional sequencing. Most importantly, this method is long and costly, since the whole gene and the flanking regions must be entirely sequenced for each patient or individual to screen.

In the approach, "Single Strand Conformation Polymorphism" (SSCP) which has been extensively used because of its simplicity, double-stranded DNA is denatured, and then renatured rapidly in conditions such that each single strand collapsed on itself. The mobility differences between the native and the mutated strands are then analysed by electrophoresis. Unfortunately, the mobility difference can vary a lot depending on the specific sequence of the DNA, and on the position of the mutation. For instance, a mutation driving DNA folding into a different path will lead to a strong difference in mobility, whereas a mutation, e.g. at the tip of a loop, will lead to essentially no change. This lack of consistency in the sensitivity makes this technique not very attractive for diagnosis, since it yields a relatively high number of false negatives. In WO 00/20853, an improved SSCP method is proposed, in which several SSCP separations with selected conditions are performed on each sample. Sensitivity is significantly improved, but the duration and complexity of the analysis is also increased considerably.

At last, it is noticed that a series of other mutation detection methods is based on the formation or dissociation of heteroduplexes.

A heteroduplex as opposed to a homoduplex is a double stranded DNA which base sequence of one strand is not entirely complementary to the base sequence of the other strand. In other words, it contains at least one base pair which is not complementary, also termed "a mismatch". A heteroduplex can be formed during DNA replication when an error is made by a DNA polymerase enzyme and a non-complementary base is added to a polynucleotide chain being replicated. A heteroduplex can also be formed during repair of a DNA lesion. Further replications of a heteroduplex will, ideally, produce homoduplexes which are heterozygous, i.e. these homoduplexes will have an altered sequence compared to the original parent DNA strand.

A majority of genetic diseases or genetic predisposition to diseases are such that affected persons have precisely for the involved gene one normal copy and one mutated copy. When a DNA fragment including the location of the gene is amplified by PCR, both genes are amplified. When the amplified DNA is denatured and then renatured slowly, 4 different types of duplex DNA are obtained, i.e.: two homoduplexes, corresponding to the DNA of the two intial alleles (the normal one and the mutated one), and two heteroduplexes, mixing one strand from one allele and the (almost) complementary strand from the other allele. These heteroduplexes contain, at the location of the mutation, a mismatch "bubble". These mismatches "bubbles" are generally searched by one of the following techniques:

In the method termed "Denaturating Gradient Gel Electrophoresis (DGGE)", duplex DNA are separated in a gel containing a gradient of denaturating conditions, so that they will melt during electrophoresis. Since the mobility of duplex and melted DNA is different, this method is very sensitive to the exact position of the melting. Heteroduplexes tend to melt faster than the corresponding homoduplexes, and can be distinguished this way. Various variants of this technique, in gel or capillary format, have been proposed, and some achieve high sensitivity. However, duplexes with different base pair contents melt at different temperatures, so that for each fragment the gradient range must be accurately adjusted. In addition, the preparation of the gradient gels is itself quite demanding and labor-intensive, so that the use of this technique for diagnosis or large scale screening is impractical.

Others techniques based on enzymatic or chemical cleavage, by e.g. cleavase, T4 endonuclease, or Osmium Tetroxyde, were also proposed. The principle of enzymatic cleavage, described e.g. in US1996/0714626 is to obtain cleavage specifically at the point of mismatch, and to analyse *in fine* the size of the fragments. High sensitivities were reported, but this technique implies extra chemical steps and, as for SSCP, the reaction is sequence dependent. A related method, described in US1994/0334612, involves recognition of the mutation by specific proteins, and analysing the resulting DNA-protein complex. This method, too, involves expensive enzymes, and the protocol is quite complex.

A third method, termed "chromatography in a gradient of denaturating conditions" (DHPLC) obeys to a principle relatively similar to that of DGGE, but the gradient of denaturating conditions is temporal, and can be automated on a HPLC apparatus. The renaturated (homoduplexes and, when relevant, heteroduplexes) DNA are adsorbed on a HPLC column, and the denaturation of DNA (which occurs slightly earlier for heteroduplexes) leads to the release of the DNA, and to detection at the output of the column (see e.g. Wagner, T., Stoppa-Lyonnet, D., Fleischmann,E., Muhr, D., Pages, S., Sandberg, T., Caux, V., Moeslinger, R., Langbauer, G., Borg, A., Oefner, P., Genomics, 1999, 62, 369-376). However, the whole process of DHPLC is long when large genes have to be screened, because of the sequential nature of HPLC, which can analyse only one sample at a time.

An improvement of the DHPLC method is proposed in WO03/031580. This invention concerns a chromatographic method for separating heteroduplex and homoduplex DNA molecules in a test mixture. In one embodiment, the method includes: (a) applying the test mixture to a reverse phase separation medium; (b) eluting the medium of step (a) with a mobile phase comprising at least one nitrogen-containing mobile phase additive, wherein the eluting is carried out under conditions effective to at least partially denature the heteroduplexes and wherein the eluting results in the separation, or at least partial separation, of the heteroduplexes from the homoduplexes. The eluting is preferably carried out at a pre-selected concentration of the additive and at a pre-selected temperature. Examples of a preferred nitrogen-containing additive include betaine, tetra methyl ammonium chloride, tetraethylammonium chloride, triethylamine hydrochloride, and choline. This improvement increases the sensitivity of DHPLC for point mutations difficult to detect, such as A/T substitutions, but it does not improve on the speed of separation, nor relieves the tedious requirement of having to adapt the denaturating gradient for each fragment to analyze.

Another method, termed "Electrophoretic Heteroduplex Analysis", (EHDA) directly measures, by electrophoresis in non-denaturating conditions, the difference in mobility induced by a « denaturation bubble » or « loop » associated with the mismatch appearing in heteroduplex pairs. When the sample is separated by electrophoresis in non-denaturing conditions, the presence of a local "mismatch bubble" on the heteroduplex molecules leads to a difference in geometry of flexibility of the double strand, which leads to a difference in mobility, as compared to the homoduplexes. If the DNA to analyse presents a normal duplex fragment and a mutated fragment (i.e. if the organism from which the DNA is extracted is heterozygote for the corresponding DNA fragment), a multiplicity of peaks (two to four) will appear in the electrophoregram. The power of the EHDA method relies mainly on the ability to detect the slight difference in migration velocity, due to the presence of mismatch bubble. This technique is simple, and fast, but up to now its detection sensitivity was weaker than that of DGGE or DHPLC, and was not considered suitable for diagnosis.

Improvement to this method has consisted to use additives such as urea, known to lower the melting point of duplex nucleic acids and increase the size of denaturation bubbles. However, such additives actually lead to a decrease in the resolution of mismatches as illustrated in the following example 5.

Another kind of improvement to EHDA involves the use of a liquid separating medium comprising block copolymer(s) with an acrylamide backbone and PDMA side-chains for electrophoretic separation of homoduplexes and heteroduplexes. The PDMA block, rather hydrophobic, is used to attach the polymer to the wall, and the backbone, hydrophilic, extends in the buffer like a "brush" and repels other macromolecules (Barbier, Fr. Pat. Appl. 00/08526 included here by reference). This allows a better resolution than conventional EHDA, and a sensitivity to mutations comparable to DHPLC. In addition, this method can be implemented in capillary array electrophoresis, thus leading to high throughput. However, in spite of this improvement, not all mutations can be detected, and further improvements are necessary.

At last, US 2002/0055109 discloses a method of isolation of heteroduplexes containing at least one internal single stranded region with a single-strand binding protein. The single-strand binding protein having a preferential affinity for single stranded DNA compared to double stranded DNA is selected from the *E*. *coli.* SSB, the product of gene 32 of phage T4, the adenovirus DBP and the calf thymus UP1. However, such a method is not suitable to detect very local mutations, such as those relative to a single base mismatch, or to an insertion or deletion of only one nucleotide or a very small number of nucleotides.

Accordingly, there is still a need for a simple and very specific method for directly detecting at least one single base difference in nucleic acids such as genomic DNA in which detection steps are minimized resulting in a method which may be performed quickly, accurately and easily with minimal operator skills. The scope of the present invention is defined by the claims.

It is precisely an object of the invention, to provide new methods for improving the detection of mutations, in particular unknown mutations, in nucleic acids.

In one of its aspects, the invention concerns a method for assaying the presence or the absence of at least one mutation on a strand of nucleic acids paired in a duplex form comprising at least the steps consisting of:
- contacting in a liquid medium said duplex, suspected to include at least one mismatch, with at least one compound able to undergo a specific base pairing interaction with said mismatch, said compound being at a concentration of at least 1g/l in said medium and being chosen from one oligonucleotide having a length of less than 5 nucleotides, a nucleotide, a base or a mixture thereof and,
- assaying for said mismatch by an analytical method.

The strands of nucleic acid paired in a duplex form are two DNA strands which are in all or in part complementary.

According to one embodiment, the method involves the use of an electrophoretic analysis as an analytical method.

According to another embodiment, the method involves the use of hybridization arrays, like DNA chips for example, as an analytical method.

According to another aspect, a method for performing Electrophoretic Heteroduplex Analysis "EHDA" on a nucleic acid sample suspected to include at least one heteroduplex said method comprising at least the steps consisting of:
- contacting in a liquid medium said nucleic acid sample suspected to include at least one heteroduplex, with at least one compound able to undergo a specific base pairing interaction with at least one mismatch of said heteroduplex, said compound being at a concentration of at least 1g/l of said medium,
- assaying for the presence of said heteroduplex thanks to its electrophoretic mobility.

In the case where the nucleic acid sample initially includes homologous strands corresponding to different alleles, the method according to the invention comprises a preliminary step of denaturating the nucleic acid sample and renaturating it in conditions convenient to achieve both heteroduplexes and homoduplexes.

In another aspect, the method involves assaying the presence or the absence of at least one mutation on a single strand of nucleic acid in a liquid medium and comprising at least the steps consisting of:
a) contacting said nucleic acid suspected to include at least one mutation with at least a nucleic acid probe grafted an a solid support,
b) allowing the hybridization of at least a part of said strand of nucleic acid with the grafted nucleic acid probe,
c) washing non-hybridized strands, and
d) assaying for said mutation by an analytical method,
wherein the steps a) and/or c) are performed in the presence of at least one compound able to undergo a specific base pairing interaction with a mismatch, said compound(s) being at a concentration of at least 1g/l.

The methods disclosed are particularly useful for either the diagnosis of the predisposition to diseases associated or putatively associated to specific point mutation(s) or the diagnosis or prognosis of such disease(s).

Accordingly, it is further related to their uses in the diagnosis of predisposition to genetic diseases or cancers or the diagnosis or prognosis of said diseases or cancers.

It is also related to the use of said methods in therapy of said diseases.

In particular, concerned diseases may include many cancers as soon as they are associated or putatively associated to specific point mutation(s) such as melanoma, ocular melanoma, leukemia, astrocytoma, glioblastoma, lymphoma, glioma, Hodgkin's lymphoma, multiple myeloma, sarcoma, myosarcoma, cholangiocarcinoma, squamous cell carcinoma, and cancers of the pancreas, breast, brain, prostate, bladder, thyroid, ovary, uterus, testis, kidney, stomach, colon and rectum.

According to another aspect, a method for assaying a nucleic acid for mutation is described comprising at least the steps consisting in:
- performing a polymerase chain reaction on said nucleic acid in the presence of at least two primers and a pool of compounds able to undergo specific base pairing interaction with nucleotides or analog thereof, said compounds being at a combined concentration of at least 1 g/l and being unable to interfere with the polymerase chain reaction and,
- analyzing and/or quantifying the so-obtained DNA fragments.

According to another aspect, composition is described including at least a compound able to undergo specific base pairing interaction at a concentration of at least 1g/l, and a pair of molecules or groups acting as a DNA probe called "molecular beacon", wherein said compound is as defined according to the invention.

In a preferred embodiment, at least one of the molecules or groups involved in the molecular beacon pair is associated with a nucleic acid or nucleic acid analog.

Also described is a composition including at least a compound able to undergo specific base pairing interaction at a concentration of at least 1g/l and at least a liquid separating medium.

In particular, a composition including at least a compound able to undergo specific base pairing interaction at a concentration of at least 1 g/l and at least a polymer compound said polymer being as defined according to the invention.

More particularly, said liquid separating medium may also include at least a compound selected among:
- a sieving polymer
- a hydrophilic polymer, and
- a surface-active polymer.

In particular, described also is a kit useful for the screening of a nucleic acid or analog thereof having a nucleic sequence related to a gene on which point mutation(s) has been associated or putatively associated with a disease or an increased predisposition to a disease, said kit comprising at least a composition as defined previously i.e. including at least a compound able to undergo specific base pairing interaction at a concentration of at least 1g/l and a polymer, as defined according to the invention.

In particular, the kit may comprise at least a compound able to undergo specific base pairing interaction at a concentration of at least 1g/l and at least a liquid separating medium, as defined according to the invention.

In particular, the kits are particularly useful for the screening of the human breast cancer predisposing genes, (BRCA) like BRCA1 and BRCA2, for mutations, notably for at least a point mutation.

According to another aspect, composition is described including at least a DNA fragment having a nucleic sequence related to a gene on which point mutations have been associated or putatively associated with a disease or an increased predisposition to a disease, and at least a compound able to undergo specific base pairing interaction at a concentration of at least 1g/l.

In particular, such a composition may include at least a DNA fragment having a nucleic sequence related to a human breast cancer predisposition gene (BRCA) and at least a compound able to undergo specific base pairing interaction at a concentration of at least 1g/l.

### COMPOUNDS ABLE TO UNDERGO SPECIFIC BASE PAIRING INTERACTIONS

According to the invention, "compound able to undergo specific base pairing interaction" in particular with a nucleotide involved in a mismatch, includes any compound presenting at least two groups suitable for hydrogen bonding, in an orientation, polarity and spacing compatible with the creation of attractive interactions with at least one of the "bases" A, T, G, U and C.

According to preferred embodiments, a compound able to undergo specific base pairing interaction with said mismatch is in particular a compound able to undergo a specific base pairing interaction with the nucleotides formed with the bases A, T, G, C, U.

In others words, the compounds considered, are capable of exhibiting an interaction specifically directed towards at least one specific base. For example, some compounds may exhibit a specific base pairing interaction for adenine, and other compounds for cytosine.

A compound may be used alone or in mixture with one or several other compounds able to undergo specific base pairing interactions with the base(s) of the nucleic acid(s) involved in the mismatch of a heteroduplex.

In a preferred embodiment, the compounds are chosen such as they cannot be incorporated into the enzymatic polymerisation of a nucleic acid, such as performed e.g. by the action of a polymerase on a single-strand template. Accordingly, said compound is unable to interfere with polymerisation reactions of nucleotides and/or to be incorporated into a newly polymerized DNA strand. By this way, said compounds are only able to enhance the detection of mutations but not prone to interfere significantly with polymerisation reactions.

The mechanism of action of the compounds considered according to the invention is very unexpected compared to that of known additives.

An interpretation of this beneficial effect, is that by performing base-pairing interactions with the denaturation bubble, the compounds of the invention stabilize the mismatch bubble, and thus increase the difference in geometry between heteroduplexes and homoduplexes. This interaction is sufficiently efficient for leading to a difference of behaviour of the heteroduplexes compared to the homoduplexes in an analytical method.

Where the analytical method is an electrophoretic method, the mobility of heteroduplexes is modified compared to homoduplexes and allows, by this way, to distinguish ones from the others. Both compounds will not have the same retention time or affinity.

Where the analytical method is a hybridization method, the induced effect is a decrease of the stability of heteroduplexes compared to homoduplexes. This difference of stability is particularly sensitive for minor mismatches, such as those resulting from substitutions, which are the most difficult mutations to detect in hybridization arrays. According to this specific embodiment, the compound(s) of the invention may be present during the hybridization and more specifically in the hybridization medium and/or during the washing step and in particular in the washing solution used for cleaning the non-hybridized strands.

Compounds interacting non-specifically with nucleic acids, such as urea or other denaturants or chaotropic solvents, also increase the probability of denaturation bubbles, but thus very likely do it also at different places along the double helix not corresponding to the mismatch, this broadening the peak in the case of electrophoretic analysis and altering the resulting separation.

In the case of the instant description, the increase in mismatch resolution is in contrast particularly significant with compounds able to undergo specific base pairing interactions with nucleotides. Very surprisingly, though, this effect is interesting mostly with compounds presenting a very limited number of base-pairing interactions (one base pairing interaction per compound is sufficient), whereas methods for mutation detection in the prior art, such as single-nucleotide primer extension, used "primers" able to achieve base pairing interactions with numerous, typically at least 12 base pairing interactions per molecule.

Another unexpected feature is that the compounds able of specific base pairing interactions with the nucleic acids to be tested, are used with best performances at much higher concentrations, as generally used for base-pairing compounds in the prior art.

In particular said compounds are used at a combined concentration of at least 1 g/l of the liquid medium used for contacting it or them with the nucleic acid to assay for mutation, preferably at least 10 g/l and most preferably at least 25 g/l.

By "combined concentration", it is understood the total concentration of said compounds, e.g. in the case of the use of several types of compounds, the combined concentration is the total of the concentrations of each compound.

The concentration of compound(s) according to the instant invention is expressed with respect to the total volume of the medium containing the nucleic acid to analyze.

According to a specific embodiment, said compound may, in addition, bear at least one substituent.

Naturally, said substituent is preferably chosen for having none significant effect or minimal effect on the amino and OH groups responsible for base genering interactions.

Such a substituent may, notably, induce in said compound at least one of the following changes:
- increase in solubility,
- change in charge, and/or
- change in friction with a solvent.

As examples of substituent that may convene to carry out the invention, one may mention substituents selected among polymeric substituents like for example polyetyhylene oxide, oligomeric substituents like for example a polyethylene glycol, hydrophilic substituent like for example dextran, water-soluble cellulose compounds, alcoholic chains, natural or synthetic peptides or polypeptides, a charged substituant like for example a quaternary ammonium and sulphate, and/or a charge.

In one embodiment of the present invention, said compounds are different from antibody.

In another embodiment, said compounds are different from enzymes.

In another embodiment, they are different from proteins.

According to a preferred embodiment, such a compound is selected in the group consisting of an oligonucleotide having a length of less than 5 nucleotides, preferably less than 3 nucleotides and more preferably less than 2 nucleotides, a nucleoside, a base or a mixture thereof.

Non restrictive examples of such compounds are:
- the bases adenine (A), guanine (G), cytosine (C), uracile (U) and thymine (T),
- adenine, guanine, cytosine, uracile and thymine bearing various substitutions, and in particular substitutions having none effect on the amino and OH groups responsible for base pairing interactions in unsubstituted bases,
- the nucleosides formed with the bases A, T, G, C, U,
- the nucleotides formed with the bases A, T, G, C, U,
- oligonucleotide analogs, and variously substituted oligonucleotides, and
- the mixtures thereof.

In numerous preferred applications, said compounds contain one single base-pairing-unit, with "base pairing unit" meaning a molecular group able to undergo one base pairing interaction with one of the bases, A, T, G, U or C.

The term "nucleotide", "oligonucleotide" or "nucleoside" is also used herein to refer to individual species or varieties of species, meaning a compound, comprising a purine or pyrimidine moiety, a ribose or deoxyribose sugar moiety, and a phosphate group, or phosphodiester linkage in the case of nucleotides within an oligonucleotide or polynucleotide.

The term "nucleotide", "oligonucleotides" and "nucleoside" is also used herein to encompass "modified species" which comprise at least one modification such as (a) an alternative linking group, (b) an analogous form of purine, (c) an analogous form of pyrimidine, or (d) an analogous sugar.

The term "nucleotide", "oligonucleotides" and "nucleoside" is also used herein to encompass "substituted species" which comprise at least one substituant chosen to increase their solubility, change their charge, or modify their friction in a solvent. For example, it may be nucleotides or nucleosides bearing a polymeric or oligomeric, hydrophilic substituant and/or a charge.

According to a particular embodiment, said compound is a nucleoside selected among the nucleosides adenosine, guanosine, uridine, cytidine, thymidine and mixtures thereof.

Preferably, said compound is cytidine at a concentration at least 1g/l, preferable at least 10 g/l, and most preferably at least 25 g/l.

Preferably, said compound is thymidine at a concentration at least 1g/l, preferable at least 10 g/l, and most preferably at least 25 g/l.

Yet more preferably, said compound is a mixture of cytidine and thymidine, each at a concentration of at least 1g/l, preferably of at least 10 g/l, and most preferably of at least 25 g/l.

It should be recognized, however, that these are only examples, convenient on the ground of availability, but that many other compounds able of undergoing specific base pairing interactions with nucleotides or nucleotide analogs can be constructed by someone skilled in the art, and enter in the frame of the invention.

In a specific embodiment, where the duplex is contacted with at least two different compounds able of undergoing specific base pairing interactions with nucleic acid, it is advantageous that these compounds cannot undergo mutually base pairing interactions, so that they remain fully available for base pairing interactions with the nucleic acids or nucleic acid analogs to be tested.

For instance, the methods according to the invention will advantageously use cytidine and thymidine, or cytidine and adenosine, or guanosine and thymidine, or guanosine and adenosine, but not e.g. thymidine and adenosine, or cytidine and guanosine.

### STRAND OF NUCLEIC ACIDS OR NUCLEIC ACID HOMOLOGS,

Strand of nucleic acids or nucleic acid homologs means single stranded RNA, DNA, LNA, PNA, or any artificial or natural analog of nucleic acids, capable of hybridizing with a natural single stranded RNA or DNA.

According to one embodiment, the methods according to the invention may be used to determine whether there is a mismatch between molecules of (1) genomic DNA or mRNA isolated from a biologic sample and (2) a nucleic acid probe complementary to wild type DNA, when molecules (1) and (2) are hybridized to each other to form a duplex.

For example, the duplex may be issued from the hybridization of BRCA1 gene genomic DNA or BRCA1 mRNA isolated from a human biologic sample with a nucleic acid probe complementary to human wild-type BRCA1 gene DNA.

Of course, within the field of the invention, the complementary strands can be in the composition as a paired or partly paired double helix.

In particular, and as a non restrictive example, in the context of the invention, "two homologous strands of nucleic acids or nucleic acids homologs" can designate a heteroduplex pair, as encountered e.g. in EHDA but also an oligonucleotide attached to a solid support, hybridized at least in part with a DNA or RNA strand contained in a solution contacted to this solid support. The latter situation is particularly suitable for application of the invention to hybridization arrays.

These strands of nucleic acid, paired in a duplex form or not, are generally issued from a nucleic acid sample.

Preferably, said nucleic acid sample contains a mixture of (+) strands and (-) strands.

Into a particular embodiment, the sample to be analysis in one run according to the method of the invention may contain nucleic acid fragments with different lengths. In a preferred embodiment, the length of each type of fragment differs from the length of any other by at least 10 bases, preferably at least 20 bases, and typically 50 bases.

When the initial nucleic acid sample contains a mixture of single stranded and double stranded nucleic acid molecules, the claimed method may comprise a preliminary and additional step of removing single stranded nucleic acid molecules.

When the initial nucleic acid sample contains mostly RNA, double stranded cDNAs are first synthesized using any technique known to those skilled in the art.Then, cDNAs of interest derived from a single gene or a limited set of genes are selectively amplified from said double stranded cDNA sample. Preferentially, methods of amplification are used to obtain targeted polynucleotide samples. Any linear or logarithmic method of amplification may be used including the ligase chain reaction, the polymerase chain reaction (PCR, RT-PCR) and techniques such as the nucleic acid sequence based amplification (NASBA).

Similarly, when the initial polynucleotide sample contains mostly genomic DNA, the targeted DNA sample is preferably obtained by PCR.

The invention encompasses all biological samples containing nucleic acid(s) without any particular limitation. More particularly, a biological sample according to the invention may originate from a cell, a tissue, an organ, a surgical or a biopsy specimen fixed or non-fixed such as bone marrow aspirates, or a biological fluid including body fluids such as whole blood, serum, plasma, cerebrospinal fluid, urine, lymph fluids, and various external secretions of the respiratory, intestinal and genito-urinary tracts, tears, saliva, milk, white blood cells, and cell culture supernatants. The origin of the sample can be animal (preferably mammal, more preferably human), plant, virus, bacteria, protozoan or fungus. The sample may be eukaryotic, prokaryotic, or acellular. Cells comprised in the biological sample, especially when coming from a tissue, organ, biological fluid or biopsy, can be cultivated in order to increase the number of available cells. The sample may contain cells from a single type or of mixed cell type. The cells, tissues and specimens may originate from normal individuals or from patient suffering from a disease or a disorder. The disease or disorder can be, for example, a cancer, a neurodegenerative disease, an inflammatory disease, a cardiovascular disease, an immune disorder, a body weight disorder such as obesity, etc. Any particular cell, cell type, pathological cell, cell at a particular state of development or disease progression, are contemplated in the present invention.

Within the scope of the invention, we call "complementary in part" two DNA, RNA, or DNA analogs, which bear complementary sequences except for one or a few mutations affecting a small number of base pairs, such as single base substitution, single base insertion or deletion, or more generally substitutions, insertions or deletions affecting only a minor fraction of the length of said DNA, RNA, or DNA analogs.

### MUTATION

The methods according to the invention are particularly advantageous for assaying point mutations.

The consequences of a point mutation may range from negligible to lethal, depending on the location and effect of the sequence change in relation to the genetic information encoded by the DNA, and it may often be at the origin of a disease or of a predisposition to a disease. The bases A and G are purines, while T and C are pyrimidines. Whereas the normal base pairings in DNA (A with T, G with C) involve one purine and one pyrimidine, the most common single base mutations involve substitution of one purine or pyrimidine for the other (e.g., A for G or C for T), a type of mutation referred to as a "transition". Mutations in which a purine is substituted for a pyrimidine, or vice versa, are less frequently occurring and are called "transversions". One may also encounter point mutations comprising the addition or loss of a single base arising in one strand of a DNA duplex at some stage of the replication process. Such mutations are called single base "insertions" or "deletions", respectively, and are also known as "frameshift" mutations, due to their effects on translation of the genetic code into proteins. Larger mutations affecting multiple base pairs also do occur and can be important in medical genetics. In particular, some mutations can arise due to "slippage" of the replication machinery in repeated DNA regions, leading to insertions or deletions of variable size.

Detectable point mutations include deletion mutation, insertion mutation, and substitution mutation wherein an incorrect base pairing occurs.

If the difference between two homologous strands of nucleic acid paired in a duplex form consists in a single nucleotide difference or a small insertion or deletion a mismatched duplex is formed. The methods according to the invention are particularly efficient for detecting mismatched duplex.

More specifically, the methods are particularly useful for the screening of a DNA fragment having a nucleic sequence related to a gene on which point mutation(s) has been associated or putatively associated with a disease or an increased predisposition to a disease. Said diseases can be different types of cancers, genetic diseases or increased predisposition to a disease such as, as an example, thalassemia, cardiovascular diseases, myopathy, cancer, and more generally genetically inheritable diseases. A non-exhaustive list of such diseases, with the associated genes, and prevalence in the population, is given in the following table as a matter of example. This list should not be considered by any means as limiting the scope of the invention, but is proposed here only to make it clear that the range of applications of the invention in human health is large and constantly expanding with the progress of genetics.

Non-limiting list of genes associated with increased predisposition to cancerous diseases, the diagnosis of which constitutes a privileged range of application of the invention.

| Predisposition | Associated putative mutated gene(s) | Frequency of mutation bearers in general population | Frequency of Frequency of in mutation affected cancer affected patients |
|---|---|---|---|
| Breast, ovary | BRCA1, BRCA2 | 1/500 | 1/30 |
| Colon, endometer (HNPCC syndrome) | hMLH1, hMSH2, hMSH6, hPMS2, TGFbeta | 1/500 | 1/20 |
| Melanoma | CDKN2A, CDK4 | 1/500 | 1/20 |
| Kidney | c-MET | 1/5000~10000 | 1/20 |
| Stomach (excluding HNPCC) | CDH1 | 1/10000~20000 | 1/100~200 |
| Colon | APC | 1/8000 | 1/100 |
| Hamartomatoses | | | |
| VHL | VHL | 1/40000 | |
| NF2 | NF2 | 1/30000 | |
| Peutz-Jegherz | LKB1 | 1/50000~100000 | |
| Gorlin syndrom | PTCH | 1/50000~100000 | |
| Cowden, Banayan-Zonana syndrom | PTEN | 1/50000~100000 | |
| NF 1 | NF1 | 1/3000 | |
| Boumeville sclerosis | TSC1,TSC2 | 1/10000~15000 | |
| Multiple endocrinian neoplasia | | | |
| Type 1 | MEN1 | 1/30000~40000 | |
| Type 2 | Ret | 1/30000~40000 | 1/10~20 |
| Carney syndrom | PRKAR1A | 1/50000~80000 | |
| DNA breakage associated diseases | | | |
| Ataxia Telangiectasia | ATM | 1/40000~300000 | |
| Fanconi disease | 6 associated genes | 1/350000 | |
| Bloom disease | BLM | 1/1000000 | |
| Xeroderma pigmentosum | 8 associated genes | 1/500000~1000000 | |
| Werner disease | WRN | 1/300000~1000000 | |

In particular, the methods are particularly useful for the screening of the human breast cancer predisposing genes, (BRCA) like BRCA1 and BRCA2, for mutations.

According to the methods described, alteration(s) of the wild type BRCA1 or BRCA2 locus may be detected. In addition, the methods can be performed by detecting the wild type BRCA1 or BRCA2 locus and thus confirming the lack of a predisposition to cancer at BRCA1 or BRCA2 locus.

As stated previously, it is also related to a composition including at least a DNA fragment having a nucleic sequence related to a gene on which point mutation(s) has been associated or putatively associated with a disease or an increased predisposition to a disease, and at least a compound able to undergo specific base pairing interaction at a concentration of at least 1 g/l, and as defined previously.

According to a specific embodiment, the nucleic sequence relates to human breast cancer predisposing genes (BRCA).

### ANALYTIC METHODS

In one embodiment of the present invention, the analytical method is an electrophoretic analysis, in particular using a liquid separating medium.

More particularly, the nucleic acid(s) having mismatch, in particular heteroduplex(es), are detected by capillary electrophoresis or electrophoresis in microchannels, in non-denaturating conditions.

The invention is particularly advantageous for capillary electrophoresis or electrophoresis in microchannels for the following reasons.

It improves significantly the sensitivity of electrophoretic analysis, especially for difficult to detect mismatches such as substitutions.

It allows for a much faster separation than sequencing.

It allows for multiplexing in different ways, thus increasing further the throughput.

In a preferred embodiment, multiplexing can be achieved by combining in the same run fragments with different lengths: since the homoduplex and heteroduplexes generally have close mobilities, several combinations of homoduplexes and heteroduplexes can be separated and identified in a single run, by mixing fragments with sufficiently different sizes. Typically, the fragments must differ in size by a factor between 10 and 100 bp, preferably between 20 and 100 bp.

In another preferred embodiment, which can be combined with the previous one, multiplexing can be performed e.g. by preparing different samples bearing tags with fluorescence at different wavelength, using e.g. PCR with different fluorescently labeled primers. The amplification of the different samples can be performed in a single reaction, and the mixture can be analysed in a single electrophoresis run. Several capillary electrophoresis machines, designed for DNA sequencing or fragment analysis, such as the ABI 310, 3100, 3700, or the Amersham "Megabace", can analyse simultaneously products with different fluorescence emission wavelength.

Finally, multiplexing can also be achieved by performing several sequential injections of different samples. The principle is the same as for the separation of fragments of different sizes: it uses the fact that, in contrast with e.g. sequencing, electrophoretic analysis of a single fragment only uses a very limited part of the separation window offered by electrophoresis, so that several samples, with start time suitable shifted, can be separated in a single run.

In another embodiment, the separation of the heteroduplex fragments can be performed by DGGE, in the presence of a composition as described in the invention, or by DHPLC in the presence of the same type of composition.

The invention also concerns a composition including at least a compound able to undergo specific base pairing interaction at a concentration of at least 1g/l and as defined previously and a polymeric liquid medium for electrophoretic analysis.

Said polymeric liquid medium includes at least a polymer useful for the separation of species.

The term "polymer" is intended to designate an ensemble of large molecules composed of smaller monomeric subunits covalently linked together in a characteristic fashion. A "homopolymer" is a polymer made up of only one kind of monomeric subunit. A "copolymer" refers to a polymer made up of two or more kinds of monomeric subunits. As used herein the term "polymer" includes homopolymers and copolymers. A "monodisperse" polymer solution means that the polymer molecules in solution have substantially equal molecular weights. Typically, a monodisperse polymer has a molecular weight distribution smaller than 1.2, and preferably smaller than 1.1. A "polydisperse" polymer solution means that the polymer molecules in solution have a significant distribution of molecular weights.

According to on embodiment, the polymers of the invention include, but not limited to N,N-disubstituted polyacrylamides, N-monosubstituted polyacrylamides, polymethacrylamide, polyvinylpyrrolidone, and the like. Exemplary substituents of the polyacrylamides includes C₁ to C₁₂ alkyl; halo-substituted C₁ to C₁₂ alkyl; methoxy-substituted C₁ to C₁₂ alkyl; hydroxyl-substituted C₁ to C₁₂ alkyl and the like. Preferably, the halo substituent is fluoro and the hydroxyl-substituted C₁ to C₁₂ alkyl is monosubstituted. It is understood that the above monomer substituents are selected so that the resulting polymer is water soluble. More preferably, exemplary substituents are selected from the group consisting of C₁ to C₃ alkyl; halo-substituted C₁ to C₃ alkyl; methoxy-substituted C₁ to C₃ alkyl; and hydroxyl-substituted C₁ to C₃ alkyl.

According to a particular embodiment, the liquid separating medium contains at least a polymer chosen in the group consisting of N,N-disubstituted polyacrylamides and N- substituted polyacrylamides, wherein said N substituents are selected from the group consisting of C₁ to C₁₂ alkyls, halo-substituted C₁ to C₁₂ alkyl, methoxy-substituted C₁ to C₁₂ alkyl, and hydroxyl-substituted C₁ to C₁₂ alkyl.

In a specific embodiment, said polymer contains acrylamide or substituted acrylamide.

Linear acrylamide is a sieving polymer for DNA electrophoresis, well-known from those skilled on the art. It is highly hydrophilic has good sieving properties but is not surface active.

According to another specific embodiment, the electrophoretic method and the composition or kit, as defined previously, involve the use of a liquid separating medium as disclosed in WO 02/01218 (included here by reference). More specifically, this liquid separating medium contains at least one polymer composed of several polymer segments, said polymer being of the irregular block copolymer type or irregular comb polymer type and having on average at least three junction points established between polymer segments of different chemical or topological nature.

One particularly preferred embodiment consists in presenting within the copolymer according to the invention at least one type of polymer segment showing, within the separating medium, specific affinity for a solid support or the channel walls, and at least one type of polymer segment showing in said medium less or no affinity for the solid support, or the channel walls.

The presence of polymer segments of this type allows the medium according to the invention to reduce the adsorption of species onto the walls of the channel and/or the electro-osmosis.

In a preferred embodiment, the liquid separation medium used according to the invention contains at least a polymer at a concentration of at least 1%, in particular of at least 3% and more particularly of at least 4% by weight of the total weight of said medium.

According to an another specific embodiment, the polymer is a block copolymer(s) having an acrylamide backbone and polydimethylacrylamide (PDMA) side-chains.

The following are most particularly suitable for the invention:
- copolymers of the comb copolymer type, the skeleton of which is of dextran, acrylamide, acrylic acid, acryloylaminoethanol or (N,N)-dimethylacrylamide type and onto which are grafted side segments of acrylamide, substituted acrylamide or (N,N)-dimethylacrylamide (DMA) type, or of the DMA/allyl glycidyl ether (AGE) copolymer type, or alternatively of homopolymer or copolymer of oxazoline or of oxazoline derivatives;
- non-thermosensitive copolymers of the irregular sequential block copolymer type having along their skeleton an alternation of segments of polyoxyethylene type and of segments of polyoxypropylene type, or an alternation of segments of polyoxyethylene type and of segments of polyoxybutylene type, or more generally an alternation of segments of polyethylene and of segments of polyether type that are appreciably more hydrophobic than polyoxyethylene;

- copolymers of the irregular sequential block copolymer type having along their skeleton an alternation of segments of acrylamide, acrylic acid, acryloylaminoethanol or dimethylacrylamide type, on the one hand, and segments of (N,N)-dimethylacrylamide (DMA) type, or of DMA/allyl glycidyl ether (AGE) copolymer type, or alternatively of homopolymer or copolymer of oxazoline or of oxazoline derivatives;
- polymers of the irregular comb polymer type, the skeleton of which is of agarose, acrylamide, substituted acrylamide, acrylic acid, acryloylaminoethanol, dimethylacrylamide (DMA), allyl glycidyl ether (AGE) polymer type, DMA/AGE random copolymer type, oxazoline and oxazoline derivative, dextran, methylcellulose, hydroxyethylcellulose, modified cellulose, polysaccharide or ether oxide type, and onto which are grafted side segments of agarose, acrylamide, substituted acrylamide, acrylic acid, acryloylaminoethanol, dimethylacrylamide (DMA), allyl glycidyl ether (AGE) polymer type, DMA/AGE random copolymer type, oxazoline and oxazoline derivative, dextran, methylcellulose, hydroxyethylcellulose, modified cellulose, polysaccharide or ether oxide type;
- copolymers of the irregular comb copolymer type, the skeleton of which is of acrylamide, substituted acrylamide, acrylic acid, acryloylaminoethanol, dimethylacrylamide (DMA), allyl glycidyl ether (AGE) polymer type, DMA/AGE random copolymer type, oxazoline and oxazoline derivative, dextran, agarose, methylcellulose, of hydroxyethylcellulose, modified cellulose, polysaccharide or ether oxide type, and bears short-chain hydrophobic side segments such as alkyl chains, aromatic derivatives, fluoroalkyls, silanes or fluorosilanes.

Additional components may also be included in particular embodiments of the liquid separating medium of the invention, such as denaturants when it is desirable to prevent the formation of duplexes or secondary structures in polynucleotides. Preferred denaturants include formamide, e.g. 40-90%, urea e.g. 6-8 M, commercially available lactams, such as pyrrolidone, and the like. Guidance for their use in electrophoresis can be found in well known molecular biology references, e.g. Sambrook et al, Molecular Cloning: A Laboratory Manual, Second Edition (Cold Spring Harbor Laboratory, New York, 1989).

It should also be noted that, in most applications, it is preferable to use a polymer according to the invention that is essentially neutral. However, it may be useful for certain applications, and in particular to avoid the adsorption of species containing both charges and hydrophobic portions, to select a polymer according to the invention that is deliberately charged, preferably opposite in charge to that of said species.

Typically, a buffer system for controlling pH may be also employed as the charge-carrying component. Exemplary buffers includes aqueous solutions of organic acids, such as citric, acetic, or formic acid; zwitterionics such as TES (N-tris[hydroxymethyl]-2-aminoethanesulfonic acid, BICINE (N,N-bis[2-hydroxyethyl]glycine, ACES (2-[2-amino-2-oxoethyl)-amino]ethanesulfonic acid), or glycylglycine; inorganic acids, such as phosphoric; and organic bases, such as Tris (Tris[hydroxymethyl]aminomethane) buffers, e.g. available from Sigma. Buffer concentration can vary widely, for example between about 1 mM to 1 M, but are typically about 20 mM. Exemplary buffer solutions for conventional capillary electrophoresis applications include the following:
(i) 0.1 M Tris, 0.25 M boric acid, 7 M urea with a pH of 7.6 for single stranded polynucleotide separations; or
(ii) 0.089 M Tris, 0.089 M boric acid, 0.005 M EDTA for double stranded polynucleotide separations. For non-zwitterionic buffer systems, preferably PDMA or polyvinylpyrrolidone are employed as the surface interaction component.

Sieving components of electrophoretic separation media may also be used.

Exemplary sieving polymers include linear polyoxides; polyethers, such as polyethylene oxide, polypropylene oxide and their copolymers; polyacrylamides; polymethacrylamide; polyvinylpyrrolidone; polyvinyloxazolidone; and a variety of water-soluble hydrophylic polymers, such as water-soluble natural gums, such as dextran; water-soluble cellulose compounds, such as methylcellulose and hydroxyethylcellulose, and copolymers and blends of these polymers. Preferably, such polymers are used at a concentration in the range between about 0.5% and 10% w:v.

As regards the preparation of the copolymers used according to the invention, it may be carried out by any conventional polymerization or copolymerization technique.

In another specific embodiment, the analytical method is a hybridization type method.

Nucleic acids probe or nucleic acid homologs may be disposed on a surface as an array of domains, such system forming a hybridization array. The invention is interesting in this context, because it enhances the difference of stability between perfectly matched pairs, and pairs with a mismatch. This enhancement is particularly sensitive for minor mismatches, such as those resulting from substitution, which are the most difficult mutation to detect in hybridization arrays.

In another specific embodiment, the nucleic acids probe or nucleic acid homologs may be disposed on the surface of particles or beads. By this way, the sample is contacted, in the presence of compounds as defined in the invention, with a multiplicity of differently tagged beads, each probe being attached to an ensemble of beads or particles bearing a unique tag or combination of tags, as described e.g. in WO 99/37814. In this method, hybridization of a single sample to a multiplicity of probes can be performed in one batch, and the sequence of the hybridized targets can be identified uniquely by the tags or the beads they bind to.

More generally, the compounds considered according to the invention are advantageous to amplify the difference between nucleic acids presenting small sequence differences, in all methods for amplification or detection of nucleic acids, involving the annealing of a primer on the target nucleic acid, or the action of a polymerase on such target nucleic acid.

According to another embodiment, the invention proposes a method for assaying a nucleic acid for mutation comprising at least the steps consisting in:
- performing a polymerase chain reaction on said nucleic acid in the presence of at least two primers and a pool of compounds able to undergo specific base pairing interaction with nucleotides or analog thereof, said compounds being at a combined concentration of at least 1 g/l and being unable to interfere with the polymerase chain reaction, and
- analyzing or quantifying the so-obtained DNA fragments.

Such a method is particularly interesting in the frame of quantitative or competitive PCR, or in the use of PCR since it is able to decrease the stability of primers on nucleic acids presenting a mismatch with the primer sequences, and thus to enhance the difference in amplification between perfectly matched and imperfectly matched amplification.

Thanks to its ability to increase the effect of mismatched base pair associated with mutations, the invention is also particularly suitable in conjunction with methods and compositions for detecting mutations using DNA probes called "molecular beacons", as described e.g. in Bonnet G, Tyagi S, Libchaber A, and Kramer FR (1999) "Thermodynamic basis of the enhanced specificity of structured DNA probes". Proc Natl Acad Sci USA 96, 6171-6176. Such DNA probes are single-stranded DNA molecules that may form a stem-and-loop structure and possess an internally quenched fluorophore. They become fluorescent only when they bind to complementary nucleic acids.

In particular, molecular beacons are combinations of a first fluorescent molecule or group and a second molecule or group capable of transferring energy to said first fluorescent molecule or group, or of quenching fluorescent from said fluorescent molecule or group, and they have been used for detection of single-nucleotide variations, as described e.g. in Marras SAE, Kramer FR, and Tyagi S (1999) "Multiplex detection of single-nucleotide variations using molecular beacons". Genet Anal 14,151-156.

Accordingly, the invention also proposes a composition including at least a compound able to undergo specific base pairing interaction as defined previously and at a concentration of at least 1g/l, and a pair of molecules or groups acting as a DNA probe called "molecular beacon".

Described is also a composition comprising a liquid separating medium as previously defined.

The liquid separating medium may include, furthermore, at least a compound selected among a sieving polymer, a hydrophilic polymer and a surface-active polymer.

The figures and examples given below are presented by way of non limiting illustration of the present invention.

### FIGURES

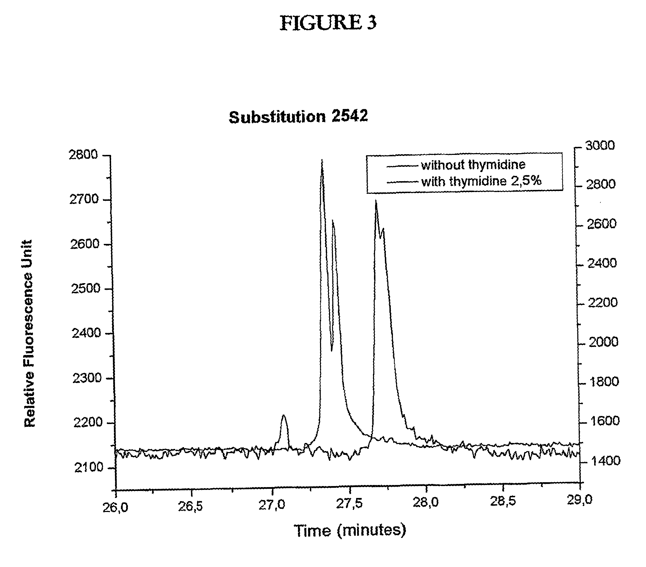

Figures 1, 2 and 3: Electropherogram representing the separation of different fragments of human genes BRCA1 and BRCA2, having respectively a different point mutation, and represented in sequence SEQ ID NO 4 (Fig. 1), SEQ ID NO 5 (Fig. 2) and SEQ ID NO 6 (Fig. 3), in presence or in absence of thymidine (see Example 1).
Figures 4 and 5: Electrophoregram representing a separation identical to that of Figure 1 involving fragments of human genes BRCA2 and BRCA1 represented in sequence SEQ ID NO 2 (Fig. 4) and SEQ ID NO 6 (Fig. 5), in presence or in absence of cytidine (see Example 2).
Figure 6: Comparison of the resolution between electrophoregram representing a separation of fragments of human genes BRCA2 represented in sequence SEQ ID NO 2, in presence or in absence of different compounds able to undergo specific base pairing interaction of the present invention (see Example 3).
Figures 7 and 8: Electrophoregram representing a separation close to that of Figures 2 and 3 in presence of different concentrations of cytidine (see Example 4).
Figure 9: Control electrophoregram representing a separation of fragments of human genes BRCA1 and BRCA2 represented in sequence SEQ ID NO 3 and in SEQ ID NO 1, in presence or in absence of urea (see Example 5).

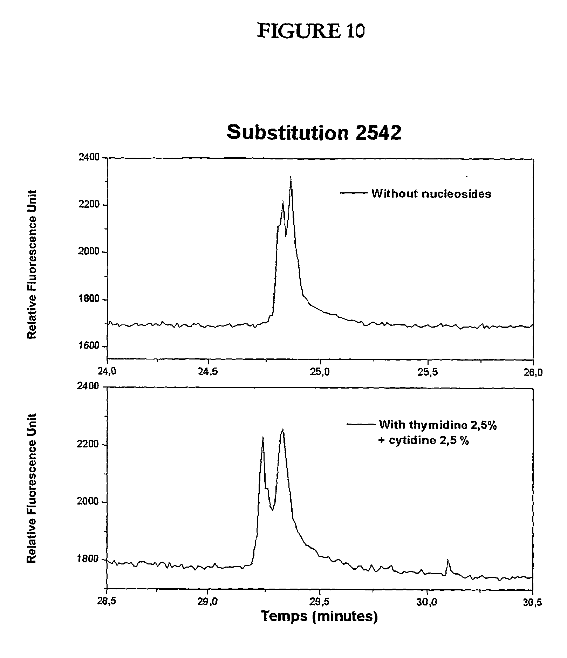

Figure 10: Electropherogram representing the separation of fragments of human genes BRCA1 represented in sequence SEQ ID NO 6, in presence or in absence of thymidine 2,5 % and cytidine 2,5 % (see Example 6).
Figure 11: Electrophoregram representing the separation a fragment of RB1 represented in SEQ ID NO 7, having a point mutation in a GC rich region.
Figure 12: Electrophoregram representing a single run with BCl/Ex 15 fragments (BRCA1) by size multiplexing.
Figure 13: Table representing SEQ ID NO 7, a CG rich region of exon of RB1 gene.
Figure 14: Table representing the primers used to amplify the sequences of the BCl/Ex 15 fragments detected according to Example 8.

### EXAMPLES

All fragments used for separations are issued from human genes BRCA1 and BRCA2.

The sequence of the genes, the primers used for amplifying the fragments are listed in table 1. Concerning the mutations termed SEQ ID NO 4 and SEQ ID NO 5 the concerned point mutation is respectively located at the eightieth and fifth place from the closest end of considered gene.

Type of substitution, fragment size, sequence (primer regions are underlined), exon and gene are shown in the following table. The point of mutation is shown in black type.

The copolymers of liquid separating medium were prepared according to the process of preparation disclosed in WO 02/01218. They have good sieving properties, and are surface-active.

| **Gene** | **Exon** | **Nucleotide position (cDNA)** | ***Identification*** | **Type** | **amplicon size (bp)** | **sequence** | **annealing t°C** |
|---|---|---|---|---|---|---|---|
| BRCA2 | 22 | 9058 | *SEQ ID NO 1 (2383)* | A/T | 311 | GTTCTGATTGCTTTTATTCCAATATCTTA AATGGTCACAGGGTTATTTCAGTGAAGA GCAGTTAAGAGCCTTGAATAATCACAGG CAAATGTTGAATGATAAGAAACAAGCTC AG A TCCAGTTGGAAATTAGGAAGGCCA TGGAATCTGCTGAACAAAAGGAACAAGG TTTATCAAGGGATGTCACAACCGTGTGG AAGTTGCGTATTGTAAGCTATTCAAAAA AAGAAAAAGATTCAGGTAAGTATGTAAA TGCTTTGTTTTTATCAGTTTTATTAACTTA AAAAATGACCTTACTAACAAAATGATTA | 58 |
| BRCA2 | 3 | 451 | *SEQ ID NO 2 (2501)* | G/C | 319 | CACTGGTTAAAACTAAGGTGGGATTTTTT TTTAAATAGATTTAGGACCAATAAGTCTT AATTGGTTTGAAGAACTTTCTTCAGAAGC TCCACCCTATAATTCTGAACCTGCAGAA GAATCTGAACATAAAAACAACAATTACG AACCAAACCTATTTAAAACTCCACAAAG GAAACCATCTTATAATCAGCTG G CTTC AACTCCAATAATATTCAAAGAGCAAGGG CTGACTCTGCCGCTGTACCAATCTCCTGT AAAAGAATAGATAAATTCAAATTAGACT TAGGTAAGTAATGCAATATGGTAGACTG GGGAGAAC | 65 |
| BRCA1 | 15 | 4719 | *SEQ ID NO 3 (1019)* | G/A | 251 | TGGCGATGGTTTTCTCCTTCCATTTATCTT TCTAGGTCATCCCCTTCTAAATGCCCATC ATTAGATGATAGGTGGTACATGCACAGT TGCTCTGGGAGTCTTCAGAATAGAAACT ACCCATCTCAAGAGGAGCTCATTAAGGT TGTTGAT G TGGAGGAGCAACAGCTGGA AGAGTCTGGGCCACACGATTTGACGGAA ACATCTTACTTGCCAAGGCAAGATCTAG GTAATATTTCATCTGCTGTATTGGA | 50 |
| BRCA1 | 15 | 4719 | *SEQ ID NO 4 (1019_80)* | G/A | 251 | GTGGTACATGCACAGTTGCTCTGGGAGT CTTCAGAATAGAAACTACCCATCTCAAG AGGAGCTCATTAAGGTTGTTGAT G TGG AGGAGCAACAGCTGGAAGAGTCTGGGCC ACACGATTTGACGGAAACATCTTACTTG CCAAGGCAAGATCTAGGTAATATTTCAT CTGCTGTATTGGAACAAACACTTTGATTT TACTCTGAATCCTACATAAAGATATTCTG GTTAACCAACTTTTAGATGTACTAGTC | 56 |
| BRCA1 | 15 | 4719 | *SEQ ID NO 5 (1019_50)* | G/A | 251 | CTTCAGAATAGAAACTACCCATCTCAAG AGGAGCTCATTAAGGTTGTTGAT G TGG AGGAGCAACAGCTGGAAGAGTCTGGGCC ACACGATTTGACGGAAACATCTTACTTG CCAAGGCAAGATCTAGGTAATATTTCAT CTGCTGTATTGGAACAAACACTTTGATTT TACTCTGAATCCTACATAAAGATATTCTG GTTAACCAACTTTTAGATGTACTAGTCTA TCATGGACACTTTTGTTATAC | 56 |
| BRCA1 | 11(01) | 855 | *SEQ ID NO 6 (2542)* | T/G | 205 | TGTATTTTTTAATGACAATTCAGTTTTT GAGTACCTTGTTATTTTTGTATATTTTCA GCTGCTTGTGAATTTTCTGAGACGGATGT AACAAATACTGAACATCATCAACCCAGT AATAATGAT T TGAACACCACTGAGAAG CGTGCAGCTGAGAGGCATCCAGAAAAGT ATCAGGGTAGTTCTGTTTCAAACTTGCAT GTGGAG | 55 |

### EXAMPLE 1

Comparison of the detection of different substitutions between:
- P(AM-PDMA)18 at 5g/100 mL + sybrgreen 1X (Molecular Probes) in Tris (50 mM) Taps (50 mM) EDTA (2 mM) buffer, and
- P(AM-PDMA)18 at 5 g/100 mL + thymidine at 2,5 g/100 mL + sybrgreen 1X in Tris (50 mM) Taps (50 mM) EDTA (2 mM) buffer.
- PDMA side-chains with initiator-monomer ratio R0=0,015 and A0=0, 01 where Ro = [R-SH]/[PDMA] and Ao = [KPS]/[PDMA] and P(AM-PDMA)18 were prepared according to the protocols presented in V. Barbier, B.A. Buchholz, A.E. Barron, J.L. Viovy, Electrophoresis, 23, 1441, (2002)) with [(NH₄)₂S₂O₈]/[AM] = 0,1 % and [Na₂S₂O₅]/[AM] = 0,015 %.

Separations were made in a ABI 310 (applied biosystem) at 30°C. Bare fused silica capillary (polymicro), 50 µm inner diameter, 61 cm long (50 cm to the detection window) were used. Injection is electrokinetic (2,5 kV during 30s). Pre-electrophoresis is done at 12,2 KV during 5 minutes. Separation is done at 12,2 kV. After each run, new polymer solution is pushed into the capillary during 10 minutes.

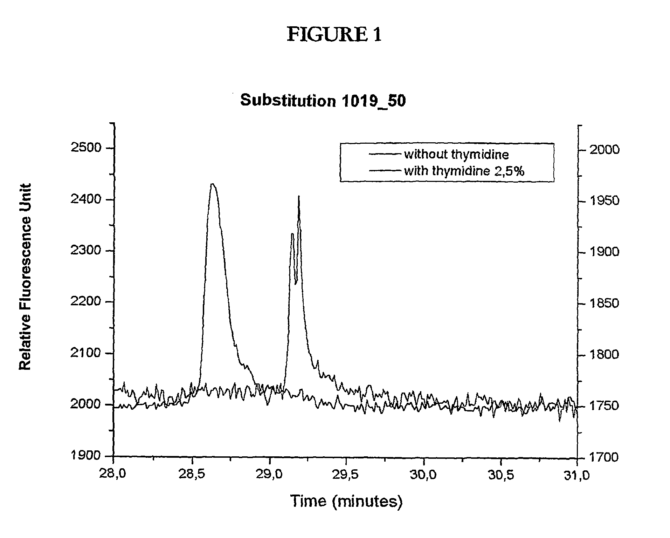

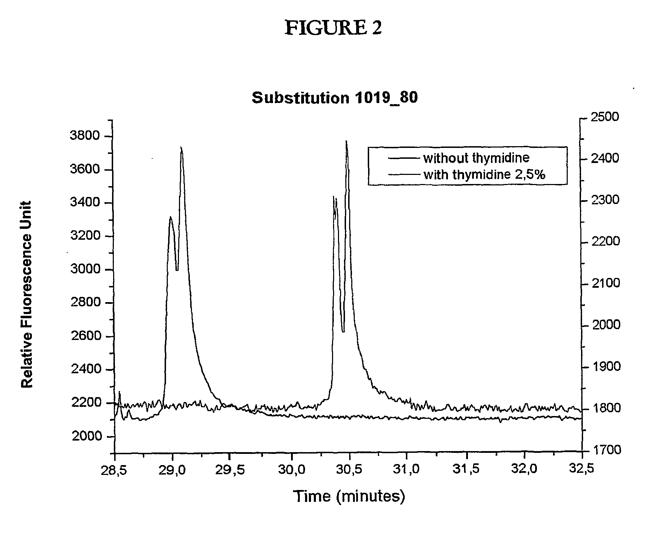

Samples were prepared by gently mixing 2 µL PCR product with 5 µL pure water (milliQ). Point mutation of SEQ ID NO 4 (Substitution 1019_80), SEQ ID NO 5 (1019_50) and SEQ ID NO 6 (2542) were studied. We can clearly observe that resolution is improved with the use of thymidine (Figure 2. and 3) and that SEQ ID NO 5 (substitution 1019_50) which was not detected with P(AM-PDMA)18 5 g/100 mL is detected with P(AM-PDMA)18 5 g/100 mL + thymidine 2,5 g/100 mL (Figure 1). For each substitution a sample from a heterozygote patient was compared to one of a homozygote. The data clearly demonstrate that the addition of thymidine, at concentrations of 2.5 g/100 ml, increase the resolution between homozygote and heterozygote DNA, and provide a clear detection of the presence of the mutation (multiple peak for the heterozygote electrophoregram).

### EXAMPLE 2

Comparison of the detection of different substitutions between:
- P(AM-PDMA)20 at 5g/100 mL + sybrgreen 1X (Molecular Probes) in Tris (50 mM) Taps (50 mM) EDTA (2 mM) buffer, and
- P(AM-PDMA)20 at 5 g/100 mL + cytidine at 2,5 g/100 mL + sybrgreen 1X in Tris (50 mM) Taps (50 mM) EDTA (2 mM) buffer.

PDMA side-chains with R0 = 0,015 and A0 = 0,01 and P(AM-PDMA)20 were prepared according to the protocols presented in (V. Barbier, B.A. Buchholz, A.E. Barron, J.L. Viovy, Electrophoresis, 23, 1441, (2002)) with [(NH₄)₂S₂O₈]/[AM] = 0,1 % and [Na₂S₂O₅]/[AM] = 0,015 %.

Separations were made in a ABI 310 (applied biosystem) at 30 °C. Bare fused silica capillary (polymicro), 50 µm inner diameter, 61 cm long (50 cm to the detection window) were used. Injection is electrokinetic (2,5 kV during 30s). Pre-electrophoresis is done at 12,2 KV during 5 minutes. Separation is done at 12,2 kV. After each run, new polymer solution is pushed into the capillary during 10 minutes.

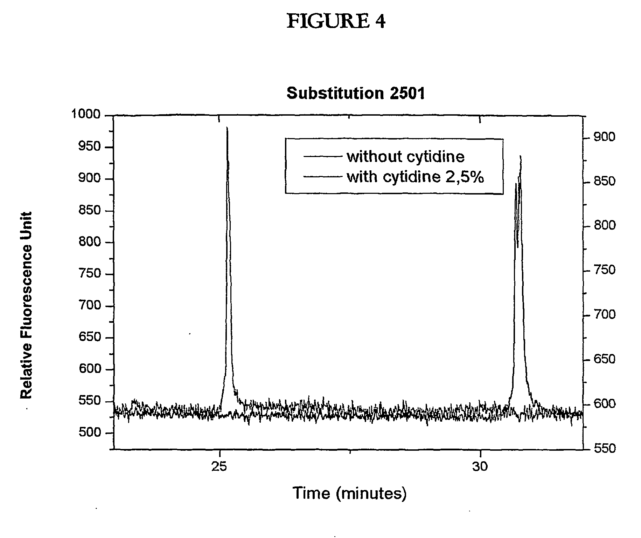

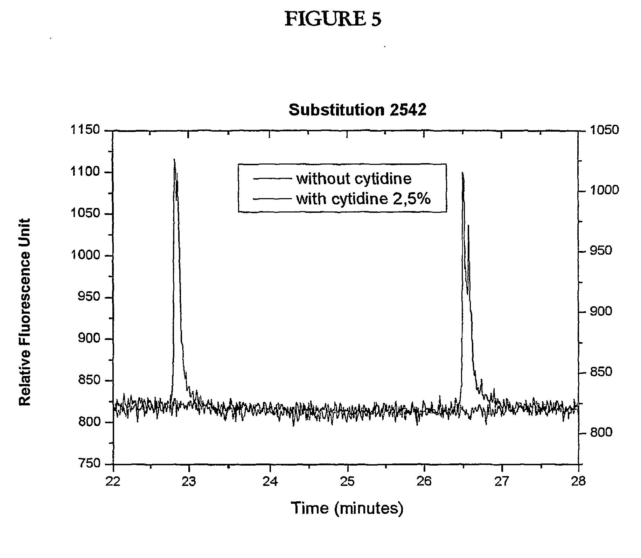

Samples were prepared by gently mixing 2 µL PCR product with 5 µL pure water (milliQ). Point mutation of SEQ ID NO 2 (Substitution 2501) and SEQ ID NO 6 (2542) were studied. Mutation of SEQ ID NO 2 (Substitution 2501) which was not detected with P(AM-PDMA)20 5 g/100 mL is detected with P(AM-PDMA)20 5 g/100 mL + cytidine 2,5 g/100 mL ( Figure 4) and resolution of mutation of SEQ ID NO 6 (substitution 2542) was improved by using P(AM-PDMA)20 5 g/100 mL + cytidine 2,5 g/100 mL compared to P(AM-PDMA)20 5 g/100 mL (Figure 5). For each substitution a sample from a heterozygote patient is compared to one of a homozygote. The data clearly demonstrate that the addition of cytidine, at concentrations of 2.5 g/100 ml, increase the resolution between homozygote and heterozygote DNA, and provide a clear detection of the presence of the mutation (multiple peak for the heterozygote electrophoregram).

### EXAMPLE 3

Comparison of the detection of different substitutions between:
- P(AM-PDMA)20 at 5g/100 mL + sybrgreen 1X (Molecular Probes) in Tris (50 mM) Taps (50 mM) EDTA (2 mM) buffer,
- P(AM-PDMA)20 at 5 g/100 mL + thymidine at 5 g/100 mL + sybrgreen 1X in Tris (50 mM) Taps (50 mM) EDTA (2 mM) buffer,
- P(AM-PDMA)20 at 5 g/100 mL + cytidine at 5 g/100 mL + sybrgreen 1X in Tris (50 mM) Taps (50 mM) EDTA (2 mM) buffer.
   And:
- P(AM-PDMA)20 at 5 g/100 mL + thymidine at 2,5 g/100 mL + cytidine at 2,5 g/100 mL + sybrgreen 1X in Tris (50 mM) Taps (50 mM) EDTA (2 mM) buffer.

Separations were made in a ABI 310 (applied biosystem) at 30°C. Bare fused silica capillary (polymicro), 50 µm inner diameter, 61 cm long (50 cm to the detection window) were used. Injection is electrokinetic (2,5 kV during 30s). Pre-electrophoresis is done at 12,2 KV during 5 minutes. Separation is done at 12,2 kV. After each run, new polymer solution is pushed into the capillary during 10 minutes.

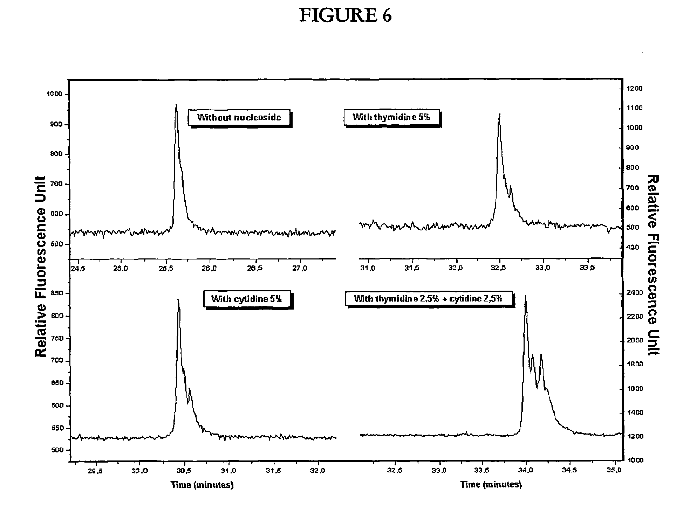

Samples were prepared by gently mixing 2 µL PCR product with 5 µL pure water (milliQ). Point mutation of SEQ ID NO 2 (Substitution 2501) was studied. This substitution is not detected with P(AM-PDMA)20 at 5 g/100 mL (only one peak). It is detected with P(AM-PDMA)20 at 5 g/100 mL + cytidine 2,5 g/100 mL and P(AM-PDMA)20 at 5 g/100 mL + thymidine 2,5 g/100 mL (two peaks) and resolution is improved with P(AM-PDMA)20 at 5 g/100 mL + cytidine 2,5 g/100 mL + thymidine 2,5 g/100 mL (three peaks) (Figure 6). For each substitution a sample from a heterozygote patient is compared to one of a homozygote. At equal concentration of nucleoside, resolution is better for the case with an equal mix of cytidine and thymidine, than with either cytidine alone, or thymidine alone. This demonstrates the synergistic effect of having in the separation medium two different additive compounds, able of achieving base pairing interactions with different nucleotides.

### EXAMPLE 4

Comparison of the detection of different substitutions between:
- P(AM-PDMA)20 at 5g/100 mL + cytidine at 0,05 g/100 mL + sybrgreen 1X in Tris (50 mM) Taps (50 mM) EDTA (2 mM) buffer,
- P(AM-PDMA)20 at 5 g/100 mL + cytidine at 1 g/100 mL + sybrgreen 1X in Tris (50 mM) Taps (50 mM) EDTA (2 mM) buffer,
- P(AM-PDMA)20 at 5 g/100 mL + cytidine at 2,5 g/100 mL + sybrgreen 1X in Tris (50 mM) Taps (50 mM) EDTA (2 mM) buffer.

Separations were made in a ABI 310 (applied biosystem) at 30°C. Bare fused silica capillary (polymicro), 50 µm inner diameter, 61 cm long (50 cm to the detection window) were used. Injection is electrokinetic (2,5 kV during 30s). Pre-electrophoresis is done at 12,2 KV during 5 minutes. Separation is done at 12,2 kV. After each run, new polymer solution is pushed into the capillary during 10 minutes.

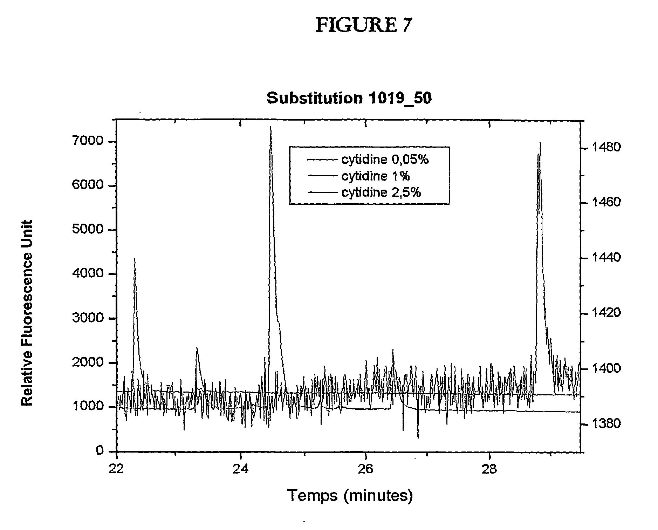

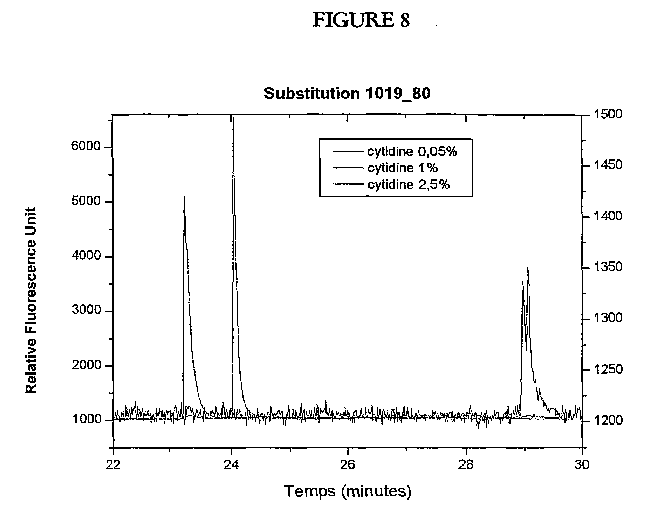

Samples were prepared by gently mixing 2 µL PCR product with 5 µL pure water (milliQ). Point mutation of SEQ ID NO 5 (Substitutions 1019_50) and SEQ ID NO 6 (1019_80) were studied. Both are not detected with a cytidine concentration inferior or equal to 1 g/100 mL (only one peak) and are detected with cytidine concentration of 2,5 g/100 mL (two peaks) (Figure 7 and 8).

### EXAMPLE 5

Comparison of the detection of different substitutions between:
- P(AM-PDMA)5 at 5g/100 mL + sybrgreen 1X in Tris (50 mM) Taps (50 mM) EDTA (2 mM) buffer,
- P(AM-PDMA)5 at 5 g/100 mL + urea at 15 g/100 mL + sybrgreen 1X in Tris (50 mM) Taps (50 mM) EDTA (2 mM) buffer,
- P(AM-PDMA)5 at 5 g/100 mL + urea at 24 g/100 mL + sybrgreen 1X in Tris (50 mM) Taps (50 mM) EDTA (2 mM) buffer.

Separations were made in a ABI 310 (applied Biosystem) at 30°C. Bare fused silica capillary (Polymicro), 50 µm inner diameter, 61 cm long (50 cm to the detection window) were used. Injection is electrokinetic (2,5 kV during 30s). Pre-electrophoresis is done at 12,2 KV during 5 minutes. Separation is done at 12,2 kV. After each run, new polymer solution is pushed into the capillary during 10 minutes.

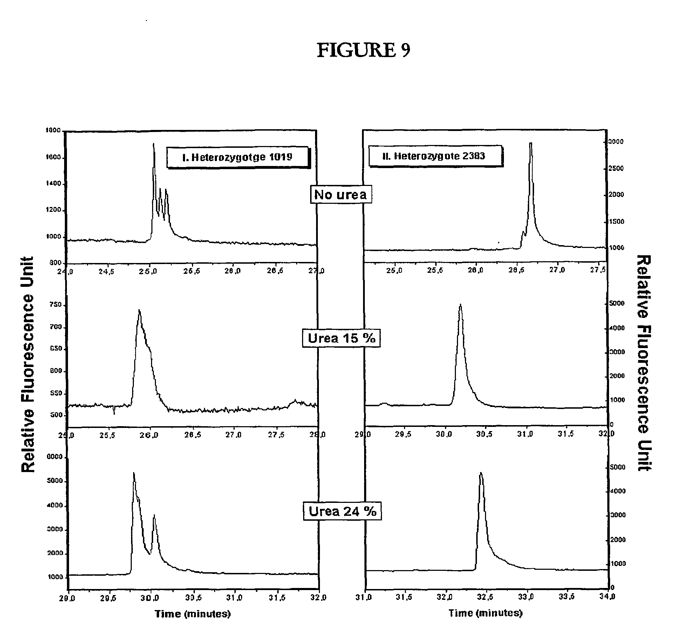

Samples were prepared by gently mixing 2 µL PCR product with 5 µL pure water (milliQ). Point mutation of SEQ ID NO 3 (Substitutions 1019) and SEQ ID NO 1 (2383) were studied. Both are detected when no urea is added to the matrix and not detected when urea concentration in the matrix is 15 g/100 mL. When urea concentration in the matrix is 24 g/100 mL, mutation of SEQ ID NO 1 (2383) is still not detected and SEQ ID NO 5 (1019) is detected with a lower resolution as in the case without urea (Figure 9). This demonstrates that additives known to favor duplex DNA denaturation but not able to lead to base pairing interactions with nucleic acids, such as urea, proposed for the resolution of mutation in the prior art, actually has an effect on the resolution of heteroduplexes which is deleterious, and opposite to this of additives of the invention.

### EXAMPLE 6

Comparison of the detection of point mutation of SEQ ID NO 6 (substitution 2542) between:
- Linear polyacrylamide at 5g/100 mL + sybrgreen 1X (Molecular Probes) in Tris (50 mM) Taps (50 mM) EDTA (2 mM) buffer, and
- Linear polyacrylamide at 5 g/100 mL + thymidine at 2,5 g/100 mL + cytidine at 2,5 g/100 mL + sybrgreen 1X in Tris (50 mM) Taps (50 mM) EDTA (2 mM) buffer.

Separations were made in a ABI 310 (Applied Biosystem) at 30°C. Bare fused silica capillary (Polymicro), 50 µm inner diameter, 61 cm long (50 cm to the detection window) was coated according the protocol presented in Chiari M, Cretich M, Horvath J. *Electrophoresis,* 2000, *21*, 1521-1526, before use. Injection is electrokinetic (2,5 kV during 30s). Pre-electrophoresis is done at 12,2 KV during 5 minutes. Separation is done at 12,2 kV. After each run, new polymer solution is pushed into the capillary during 10 minutes.

Samples were prepared by gently mixing 2 µL PCR product with 5 µL pure water (milliQ). The figure 6 shows clearly observe that resolution is improved with the use of nucleosides. A sample from a heterozygote patient was compared to one of a homozygote.

### EXAMPLE 7

Detection of a substitution in a GC rich region of exon 8 of RB1 gene.

This mutation, of the type of a C->T transition (nomenclature code g 59695 C->T/arg 255 stop) introduces a stop codon in a GC rich region of the gene, and is thus a deleterious mutation. It is a highly recurrent mutation, and thus important to detect for diagnosis of retinoblastoma, but it is not detected in DHPLC.

Separations were made with P(AM-PDMA) at 5 g/100 mL + thymidine at 2,5 g/100 mL + cytidine at 2,5 g/100 mL + sybrgreen 1X in Tris (50mM) Taps (50 mM) EDTA (2 mM) buffer in a ABI 310 applied biosystem) at 30°C. Bare fused silica capillary (polymicro), 50 µm inner diameter, 61 cm long (50 cm to the detection window) were used. Injection is electrokinetic (2,5 kV during 30 s). Pre-electrophoresis is done at 12,2 kV during 5 minutes. Separation is done at 15 kV. After each run, new polymer solution is pushed into the capillary during 10 minutes.

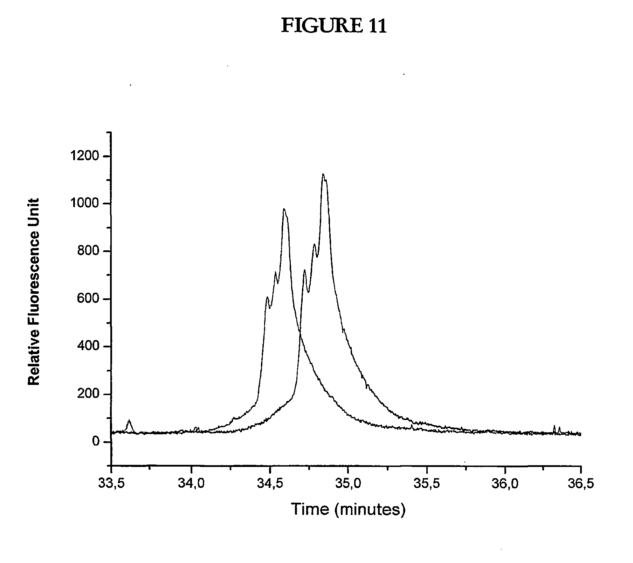

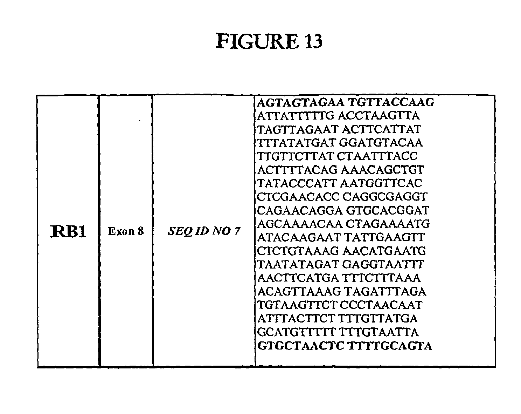

Samples were prepared by gently mixing 2 µL PCR product with 5 µL pure water (milliQ). Figure 11 shows detection of the mutation for 2 successive runs. Figure 13 shows sequence of the amplicon with primers indicated in bold letters (SEQ ID NO 7).

### EXAMPLE 8

Size multiplexing allows the detection of multiple mutations in the same run.

Separations were made with P(AM-PDMA) at sag/100 mL + sybrgreen 1X in Tris (50 mM) Taps (50 mM) EDTA (2 mM) buffer in a ABI 310 (applied biosystem) at 30°C. Bare fused silica capillary (polymicro), 50 µm inner diameter, 61 cm long (50 cm to the detection window) were used. Injection is electrokinetic (2,5 kV during 30s). Pre-electrophoresis is done at 12,2 KV during 5 minutes. Separation is done at 15 kV. After each run, new polymer solution is pushed into the capillary during 10 minutes.

Samples were prepared by gently mixing 2 µL PCR product with 5 µL pure water (milliQ).

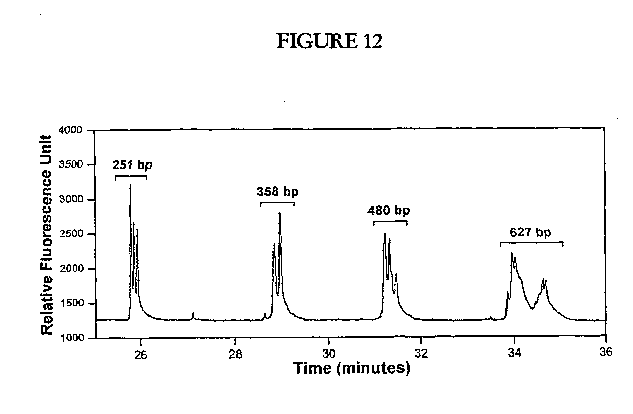

Figure 12 represents a single run with BCl/Ex15 fragments (BRCA1 gene) sizing 254 bp, 358 bp, 480 bp and 627 was performed in a 50 cm effective length, 50 µm i.d capillary at 30°C and 200 V/cm. The mutation is the same for all fragments, only the size differs from one peak to the other. All mutations were detected.

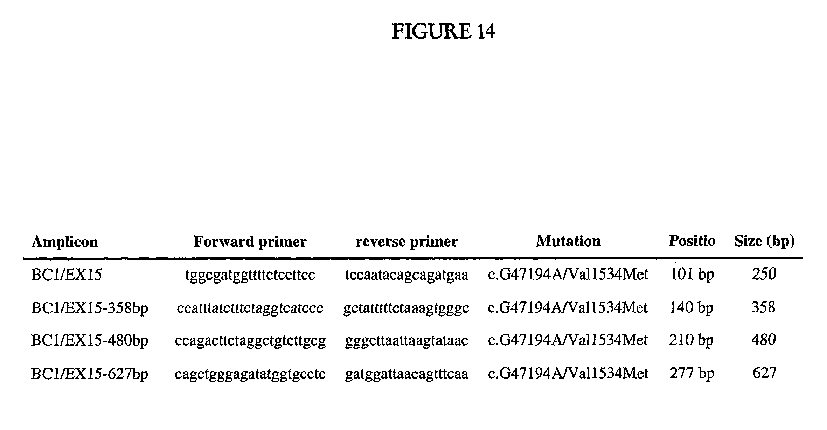

The characteristics of the fragments are given in the Figure 14.

## Claims

1. A method for assaying the presence or the absence of at least one mutation on a strand of nucleic acids paired in a duplex form comprising at least the steps consisting of:
- contacting in a liquid medium said duplex, suspected to include at least one mismatch with at least one compound able to undergo a specific base pairing interaction with said mismatch, said compound(s) being used at a combined concentration of at least 10g/l in said medium and being chosen from one oligonucleotide having a length of less than 5 nucleotides, a nucleoside, a base or a mixture thereof and,
- assaying for said mismatch by an analytical method.

2. The method according to claim 1, wherein the strands of nucleic acids paired in duplex form are two DNA strands which are in all or in part complementary.

3. A method for performing Electrophoretic Heteroduplex Analysis "EHDA" on a nucleic acid sample suspected to include at least one heteroduplex, said method comprising at least the steps consisting of:
- contacting in a liquid medium said nucleic acid sample suspected to include at least one heteroduplex, with at least one compound able to undergo a specific base pairing interaction with at least one mismatch of said heteroduplex, said compound(s) being used at a combined concentration of at least 10 g/l of said medium, and being chosen from one oligonucleotide having a length of less than 5 nucleotides, a nucleoside, a base or a mixture thereof and,
- assaying for the presence of said heteroduplex thanks to its electrophoretic mobility.

4. The method according to claim 3 comprising a preliminary step of denaturating the nucleic acid sample and renaturating it in conditions convenient to achieve both heteroduplexes and homoduplexes.

5. A method for assaying the presence or the absence of at least one mutation on a single strand of nucleic acid in a liquid medium comprising at least the steps consisting of:
(a) contacting said nucleic acid suspected to include at least one mutation with a nucleic acid probe grafted on a solid support,
(b) allowing the hybridization of at least a part of said strand of nucleic acid with the grafted nucleic acid probe,
(c) washing non-hybridized strands, and
(d) assaying for said mutation by an analytical method,
wherein the steps a) and/or c) are performed in the presence of at least one compound able to undergo a specific base pairing interaction with said mutation, said compound being at a concentration of at least 1g/l and being chosen from one oligonucleotide having a length of less than 5 nucleotides, a nucleoside, a base or a mixture thereof.

6. The method according to claims 1 to 5 wherein the strand(s) of nucleic acids is a single stranded DNA, RNA, LNA, PNA, or any artificial or natural analog of nucleic acids.

7. The method according to any one of claims 1 to 6, wherein the compound able to undergo a specific base pairing interaction includes at least two groups suitable for hydrogen bonding, in an orientation, polarity and spacing compatible with the creation of attractive interaction with at least one of the bases A, T, G, C and U.

8. The method according to any one of claims 1 to 7, wherein said compound is unable to interfere with polymerisation reactions of nucleotides and/or to be incorporated into a newly polymerized DNA strand.

9. The method according to any one of the preceding claims, wherein the oligonucleotide has a length of less than 3 nucleotides and preferably less than 2 nucleotides.

10. The method according to any one of the preceding claims, wherein the compound is selected among adenosine, guanosine, uridine, cytidine, thymidine and mixtures thereof.

11. The method according to any one of the preceding claims, wherein oligonucleotide(s) or nucleoside(s) in the mixture of oligonucleotides or nucleosides are unable to undergo mutually base pairing interaction.

12. The method according to claim 11, wherein the compound includes cytidine and thymidine or cytidine and adenosine or guanosine and thymidine or guanosine and adenosine.

13. The method according to any one of the claims 5 to 12, wherein the compound(s) is used at a concentration of a least 10 g/l.

14. The method according to any one of the claims 1 to 13, wherein the compound(s) is used at a concentration of at least 25 g/l.

15. The method according to any one of claims 1 to 14, in which said compound(s) is in addition bearing at least one substituent.

16. The method according to claim 15, in which said substituent induces in said compound at least one of the following changes:
- increase in solubility,
- change in charge, or
- change in friction with a solvent.

17. The method according to any one of the claims 1 to 16, wherein the mutation to assay is a point mutation.

18. The method according to any one of the claims 1 or 2 and 5 to 17, wherein said mutation is assayed by hybridization assay.

19. The method according to any one of the claims 1 to 4 or 6 to 17, wherein said mutation is assayed by an electrophoretic analysis using a liquid separating medium.

20. The method according to claim 19, wherein said liquid separating medium contains at least a polymer at a concentration of at least 1%, and preferably at least 3% by weight of the total weight of said medium.

21. The method according to claim 19 or 20, wherein said liquid separating medium contains at least a polymer chosen in the group consisting of N,N-disubstituted polyacrylamides and N- substituted polyacrylamides, wherein said N substituents are selected from the group consisting of C₁ to C₁₂ alkyl, halo-substituted C₁ to C₁₂ alkyl, methoxy-substituted C₁ to C₁₂ alkyl, and hydroxyl-substituted C₁ to C₁₂ alkyl.

22. The method according to claim 20 or 21, wherein the liquid separation medium contains at least one polymer composed of several polymer segments, said polymer being of the irregular block copolymer type or irregular comb polymer type and having on average at least three junction points established between polymer segments of different chemical or topological nature.

23. The method according to claim 22, wherein the polymer comprises at least one type of polymer segment showing, within the separating medium, specific affinity for the channel wall, and at least one type of polymer segment showing in said medium less or no affinity for the wall.

24. The method according to claim 20 to 23, wherein said polymer contains acrylamide or substituted acrylamides.

25. Use of a method according to any one of the claims 1 to 24 for diagnosing a predisposition to genetic diseases or cancers associated or putatively associated to specific point mutation(s) or the diagnosis or prognosis of said diseases or cancers.

26. The use according to claim 25, wherein said disease is associated to at least a point mutation in a human breast cancer predisposition gene (BRCA).

27. Use of a composition including at least a compound able to undergo specific base pairing interaction said compound being present at a concentration of at least 1 g/l and being chosen from one oligonucleotide having a length of less than 5 nucleotides, a nucleoside, a base or a mixture thereof and, at least a liquid separating medium for assaying the presence or the absence of at least one mutation on a strand of nucleic acids paired in a duplex form.

28. The use according to claim 27, wherein the compound is as defined in any one of the claims 7 to 16.

29. The use according to claim 27 or claim 28, wherein said liquid separating medium is a defined in claims 20 to 24.

30. The use according to any one of claims 27 to 29, wherein said liquid separation medium includes furthermore at least a compound selected among:
- a sieving polymer
- a hydrophilic polymer, and
- a surface-active polymer.

31. Use of a composition including at least a DNA fragment having a nucleic sequence related to a gene on which point mutation(s) has been associated or putatively associated with a disease or an increased predisposition to a disease, and at least a compound able to undergo specific base pairing interaction, said compound(s) being used at a combined concentration of at least 10 g/l and being chosen from one oligonucleotide having a length of less than 5 nucleotides, a nucleoside, a base or a mixture thereof for assaying the presence or the absence of at least one mutation on a strand of nucleic acids paired in a duplex form.

32. Use of a composition including at least a compound able to undergo specific base pairing interaction said compound being present at a concentration of at least 1g/l and being chosen from one oligonucleotide having a length of less than 5 nucleotides, a nucleoside, a base or a mixture thereof, and a pair of molecules or groups acting as a DNA probe called "molecular beacon" for assaying the presence or the absence of at least one mutation on a strand of nucleic acids paired in a duplex form.

33. Use according to anyone of claims 27 to 32, for carrying out a method according to anyone of claims 1 to 24.

34. A method for assaying a nucleic acid for mutation comprising at least the steps consisting in:
- performing a polymerase chain reaction on said nucleic acid in the presence of at least two primers and a pool of compounds able to undergo specific base pairing interaction with nucleotides or analogue thereof, said compounds being present at a combined concentration of at least 10 g/l, being chosen from one oligonucleotide having a length of less than 5 nucleotides, a nucleoside, a base or a mixture thereof and, being unable to interfere with the polymerase chain reaction and,
- analyzing and/or quantifying the so-obtained DNA fragments.

35. The method according to claim 33, wherein the compound is as defined in any one of the claims 7 to 16.

## Patentansprüche

1. Verfahren zum Bestimmen der Anwesenheit oder Abwesenheit mindestens einer Mutation auf einem Strang von in Duplexform gepaarten Nukleinsäuren, welches mindestens die folgenden Stufen umfasst:
- das Kontaktieren des Duplexes, das mutmaßlich mindestens eine Fehlpaarung aufweist, in einem flüssigen Medium mit mindestens einer Verbindung, die mit der Fehlpaarung eine spezifische Basenpaarungs-Wechselwirkung eingehen kann, wobei die Verbindung(en) in einer vereinten Konzentration von mindestens 10 g/l in dem Medium eingesetzt und unter einem Oligonukleotid einer Länge von weniger als 5 Nukleotiden, einem Nukleosid, einer Base oder einem Gemisch davon ausgewählt wird/werden, und
- die Bestimmung der Fehlpaarung durch ein analytisches Verfahren.

2. Verfahren nach Anspruch 1, wobei die Stränge der Nukleinsäuren, die in Duplexform gepaart sind, zwei DNA-Stränge sind, die vollständig oder teilweise komplementär sind.

3. Verfahren zum Durchführen einer elektrophoretischen Heteroduplexanalyse "EHDA" einer Nukleinsäureprobe, die mutmaßlich ein Heteroduplex aufweist, wobei das Verfahren mindestens die folgenden Stufen umfasst:
- das Kontaktieren der Nukleinsäureprobe, die mutmaßlich mindestens ein Heteroduplex enthält, in einem flüssigen Medium mit mindestens einer Verbindung, die mit mindestens einer Fehlpaarung des Heteroduplexes eine spezifische Basenpaarungs-Wechselwirkung eingehen kann, wobei die Verbindung(en) in einer vereinten Konzentration von mindestens 10 g/l des Mediums eingesetzt und unter einem Oligonukleotid einer Länge von weniger als 5 Nukleotiden, einem Nukleosid, einer Base oder einem Gemisch davon ausgewählt wird/werden, und
- das Bestimmen der Anwesenheit des Heteroduplexes aufgrund seiner elektrophoretischen Mobilität.

4. Verfahren nach Anspruch 3, welches eine vorbereitende Stufe umfasst, in der die Nukleinsäureprobe denaturiert wird und sie unter Bedingungen, unter denen sowohl Heteroduplexe als auch Homoduplexe bequem erhalten werden können, renaturiert wird.

5. Verfahren zum Bestimmen der Anwesenheit oder der Abwesenheit mindestens einer Mutation auf einem Einzelstrang einer Nukleinsäure in einem flüssigen Medium, welches mindestens die folgenden Stufen umfasst:
(a) das Kontaktieren der Nukleinsäure, die mutmaßlich mindestens eine Mutation aufweist, mit einer auf einem festen Träger aufgepfropften Nukleinsäuresonde,
(b) das Zulassen der Hybridisierung mindestens eines Teils des Stranges der Nukleinsäure mit der aufgepfropften Nukleinsäuresonde,
(c) das Abwaschen von nicht hybridisierten Stränge, und
(d) das Bestimmen der Mutation durch ein analytisches Verfahren,
wobei die Stufen a) und/oder c) in Anwesenheit mindestens einer Verbindung durchgeführt wird/werden, die mit der Mutation eine spezifische Basenpaarungs-Wechselwirkung eingehen kann, wobei die Verbindung in einer Konzentration von mindestens 1 g/l enthalten ist und unter einem Oligonukleotid einer Länge von weniger als 5 Nukleotiden, einem Nukleosid, einer Base oder einem Gemisch davon ausgewählt ist.

6. Verfahren nach den Ansprüchen 1 bis 5, wobei der Strang oder die Stränge der Nukleinsäuren einzelsträngige DNA, RNA, LNA, PNA oder ein beliebiges künstliches oder natürliches Analogen von Nukleinsäuren ist/sind.

7. Verfahren nach einem der Ansprüche 1 bis 6, wobei die Verbindung, die eine spezifische Basenpaarungs-Wechselwirkung eingehen kann, mindestens zwei für Wasserstoffbrückenbindungen geeignete Gruppen in mit der Ausbildung einer anziehenden Wechselwirkung mit mindestens einer der Basen A, T, G, C und U kompatiblen Ausrichtung, Polarität und Abstand umfasst.

8. Verfahren nach einem der Ansprüche 1 bis 7, wobei die Verbindung Polymerisationsreaktionen von Nukleotiden nicht beeinträchtigen kann und/oder nicht in einen neu polymerisierten DNA-Strang eingebaut werden kann.

9. Verfahren nach einem der Ansprüche 1 bis 8, wobei das Oligonukleotid eine Länge von weniger als drei Nukleotiden und vorzugsweise weniger als 2 Nukleotiden hat.

10. Verfahren nach einem der vorstehenden Ansprüche, wobei die Verbindung unter Adenosin, Guanosin, Uridin, Cytidin, Thymidin und Gemischen davon ausgewählt ist.

11. Verfahren nach einem der vorstehenden Ansprüche, wobei das Oligonukleotid oder die Oligonukleotide oder das Nukleosid oder die Nukleoside in dem Gemisch von Oligonukleotiden oder Nukleosiden keine gegenseitigen Basenpaarungs-Wechselwirkungen eingehen können.

12. Verfahren nach Anspruch 11, wobei die Verbindung Cytidin und Thymidin oder Cytidin und Adenosin oder Guanosin und Thymidin oder Guanosin und Adenosin enthält.

13. Verfahren nach einem der Ansprüche 5 bis 12, wobei die Verbindung(en) in einer Konzentration von mindestens 10 g/l eingesetzt wird/werden.

14. Verfahren nach einem der Ansprüche 1 bis 13, wobei die Verbindung(en) in einer Konzentration von mindestens 25 g/l eingesetzt wird/werden.

15. Verfahren nach einem der Ansprüche 1 bis 14, wobei die Verbindung(en) zusätzlich mindestens einen Substituenten aufweist/aufweisen.

16. Verfahren nach Anspruch 15, wobei der Substituent in der Verbindung mindestens eine der folgenden Änderungen induziert:
- Erhöhung der Löslichkeit,
- Änderung der Ladung oder
- Änderung der Reibung mit einem Lösungsmittel.

17. Verfahren nach einem der Ansprüche 1 bis 16, wobei die zu bestimmende Mutation eine Punktmutation ist.

18. Verfahren nach einem der Ansprüche 1 oder 2 und 5 bis 17, wobei die Mutation durch Hybridisierung bestimmt wird.

19. Verfahren nach einem der Ansprüche 1 bis 4 oder 6 bis 17, wobei die Mutation durch elektrophoretische Analyse unter Einsatz eines flüssigen Trennmediums bestimmt wird.

20. Verfahren nach Anspruch 19, wobei das flüssige Trennmedium mindestens ein Polymer in einer Konzentration von mindestens 1% und vorzugsweise mindestens 3 Gew.-% des Gesamtgewichts des Mediums enthält.

21. Verfahren nach Anspruch 19 oder 20, wobei das flüssige Trennmedium mindestens ein Polymer enthält, das aus der Gruppe ausgewählt ist, die aus N,N-disubstituierten Polyacrylamiden und N-substituierten Polyacrylamiden besteht, wobei die N-Substituenten aus der Gruppe ausgewählt sind, die aus C₁- bis C₁₂-Alkyl, halogensubstituiertem C₁- bis C₁₂-Alkyl, methoxysubstituiertem C₁- bis C₁₂-Alkyl und hydroxysubstituiertem C₁- bis C₁₂-Alkyl besteht.

22. Verfahren nach Anspruch 20 oder 21, wobei das flüssige Trennmedium mindestens ein Polymer enthält, das aus mehreren Polymersegmenten zusammengesetzt ist, wobei das Polymer vom unregelmäßigen Blockcopolymer-Typ oder vom unregelmäßigen Kammpolymer-Typ ist und im Mittel mindestens drei Verknüpfungspunkte zwischen Polymersegmenten unterschiedlicher chemischer oder topologischer Art aufweist.

23. Verfahren nach Anspruch 22, wobei das Polymer mindestens einen Typ von Polymersegmenten, der mit dem Trennmedium eine spezifische Affinität für die Kanalwand zeigt, und mindestens einen Typ eines Polymersegments aufweist, der in dem Medium weniger oder keine Affinität für die Wand zeigt.

24. Verfahren nach Anspruch 20 bis 23, wobei das Polymer Acrylamid oder substituierte Acrylamide enthält.

25. Verwendung eines Verfahrens nach einem der Ansprüche 1 bis 24 für die Diagnose einer Prädisposition für genetische Krankheiten oder Krebsarten, die mit einer oder mehreren spezifischen Punktmutationen assoziiert oder mutmaßlich assoziiert sind, oder für die Diagnose oder Vorhersage dieser Krankheiten oder Krebsarten.

26. Verwendung nach Anspruch 25, wobei die Krankheit mit mindestens einer Punktmutation in einem menschlichen Brustkrebs-Prädispositionsgen (BRCA) in Verbindung steht.

27. Verwendung einer Zusammensetzung, die mindestens eine Verbindung, die eine spezifische Basenpaarungs-Wechselwirkung eingehen kann, wobei die Verbindung in einer Konzentration von mindestens 1 g/l enthalten ist und unter einem Oligonukleotid einer Länge von weniger als 5 Nukleotiden, einem Nukleosid, einer Base oder einem Gemisch davon ausgewählt ist, und mindestens ein flüssiges Trennmedium zum Bestimmen der Anwesenheit oder Abwesenheit mindestens einer Mutation auf einem Strang von in Duplexform gepaarten Nukleinsäuren enthält.

28. Verwendung nach Anspruch 27, wobei die Verbindung wie in einem der Ansprüche 7 bis 16 definiert ist.

29. Verwendung nach Anspruch 27 oder Anspruch 28, wobei das flüssige Trennmedium wie in den Ansprüchen 20 bis 24 definiert ist.

30. Verwendung nach einem der Ansprüche 27 bis 29, wobei das flüssige Trennmedium außerdem mindestens eine der folgenden Verbindungen enthält:
- ein Siebpolymer,
- ein hydrophiles Polymer und
- ein oberflächenaktives Polymer.

31. Verwendung einer Zusammensetzung, die mindestens ein DNA-Fragment mit einer Nukleinsequenz, die sich auf ein Gen bezieht, auf dem ein oder mehrere Punktmutationen mit einer Krankheit oder einer erhöhten Prädisposition für eine Krankheit assoziiert oder mutmaßlich assoziiert sind, und mindestens eine Verbindung enthält, die eine spezifische Basenpaarungs-Wechselwirkung eingehen kann, wobei die Verbindung(en) in einer vereinten Konzentration von mindestens 10 g/l eingesetzt und unter einem Oligonukleotid einer Länge von weniger als 5 Nukleotiden, einem Nukleosid, einer Base oder einem Gemisch davon ausgewählt ist, für die Bestimmung der Anwesenheit oder Abwesenheit mindestens einer Mutation auf einem Strang von in Duplexform gepaarten Nukleinsäuren.

32. Verwendung einer Zusammensetzung, die mindestens eine Verbindung, die eine spezifische Basenpaarungs-Wechselwirkung eingehen kann, wobei die Verbindung in einer Konzentration von mindestens 1 g/l vorhanden ist und unter einem Oligonukleotid einer Länge von weniger als 5 Nukleotiden, einem Nukleosid, einer Base oder einem Gemisch davon ausgewählt ist, und ein als DNA-Sonde wirkendes und als "molekulares Leuchtfeuer" bezeichnetes Paar von Molekülen oder Gruppen enthält, für die Bestimmung der Anwesenheit oder Abwesenheit mindestens einer Mutation auf einem Strang von in Duplexform gepaarten Nukleinsäuren.

33. Verwendung nach einem der Ansprüche 27 bis 32 zum Durchführen eines Verfahrens nach einem der Ansprüche 1 bis 24.

34. Verfahren zum Bestimmen einer Nukleinsäure für die Mutation, welches mindestens die folgenden Stufen umfasst:
- das Durchführen einer Polymerasekettenreaktion auf der Nukleinsäure in Anwesenheit von mindestens zwei Primern und einer Gruppe von Verbindungen, die mit Nukleotiden oder Analogen davon eine spezifische Basenpaarungs-Wechselwirkung eingehen können, wobei die Verbindungen in einer vereinten Konzentration von mindestens 10 g/l vorhanden sind, unter einem Oligonukleotid einer Länge von weniger als 5 Nukleotiden, einem Nukleosid, einer Base oder einem Gemisch davon ausgewählt sind und die Polymerasekettenreaktion nicht beeinträchtigen, und
- das Analysieren und/oder Quantifizieren der so erhaltenen DNA-Fragmente.

35. Verfahren nach Anspruch 33, wobei die Verbindung wie in einem der Ansprüche 7 bis 16 definiert ist.

## Revendications

1. Méthode pour détecter la présence ou l'absence d'au moins une mutation sur un brin d'acides nucléiques appariés sous la forme d'un duplex comprenant au moins les étapes consistant à :
- mettre en contact dans un milieu liquide ledit duplex, soupçonné de contenir au moins un mésappariement avec au moins un composé capable de subir une interaction d'appariement de bases spécifique avec ledit mésappariement, le(s)dit(s) composé(s) étant utilisé(s) à une concentration combinée d'au moins 10 g/l dans ledit milieu et étant choisi(s) parmi un oligonucléotide ayant une longueur de moins de 5 nucléotides, un nucléoside, une base ou un mélange de ceux-ci et,
- détecter ledit mésappariement par une méthode analytique.

2. Méthode selon la revendication 1, dans laquelle les brins des acides nucléiques appariés sous la forme d'un duplex sont deux brins d'ADN qui sont totalement ou en partie complémentaires.

3. Méthode pour réaliser une analyse électrophorétique d'hétéroduplex « EHDA » sur un échantillon d'acides nucléiques soupçonné de contenir au moins un hétéroduplex, ladite méthode comprenant au moins les étapes consistant à :
- mettre en contact dans un milieu liquide ledit échantillon d'acides nucléiques soupçonné de contenir au moins un hétéroduplex, avec au moins un composé capable de subir une interaction d'appariement de bases spécifique avec au moins un mésappariement dudit hétéroduplex, le(s)dit(s) composé(s) étant utilisé(s) à une concentration combinée d'au moins 10 g/l dans ledit milieu, et étant choisi(s) parmi un oligonucléotide ayant une longueur de moins de 5 nucléotides, un nucléoside, une base ou un mélange de ceux-ci et,
- détecter la présence dudit hétéroduplex grâce à sa mobilité électrophorétique.

4. Méthode selon la revendication 3 comprenant une étape préliminaire de dénaturation de l'échantillon d'acides nucléiques et sa renaturation dans des conditions appropriées pour obtenir à la fois des hétéroduplex et des homoduplex.

5. Méthode pour détecter la présence ou l'absence d'au moins une mutation sur un simple brin d'acide nucléique dans un milieu liquide comprenant au moins les étapes consistant à :
a) mettre en contact ledit acide nucléique soupçonné de contenir au moins une mutation avec une sonde d'acide nucléique greffée sur un support solide,
b) permettre l'hybridation d'au moins une partie dudit brin d'acide nucléique avec la sonde d'acide nucléique greffée,
c) laver les brins non hybrides, et
d) rechercher ladite mutation par une méthode analytique,
dans laquelle les étapes a) et/ou c) sont réalisées en présence d'au moins un composé capable de subir une interaction d'appariement de bases spécifique avec ladite mutation, ledit composé étant à une concentration d'au moins 1 g/l et étant choisi parmi un oligonucléotide ayant une longueur de moins de 5 nucléotides, un nucléoside, une base ou un mélange de ceux-ci.

6. Méthode selon les revendications 1 à 5, dans laquelle le(s) brin(s) d'acides nucléiques est (sont) un ADN simple brin, ARN, LNA, PNA ou tout analogue artificiel ou naturel d'acides nucléiques.

7. Méthode selon l'une quelconque des revendications 1 à 6, dans laquelle le composé capable de subir une interaction d'appariement de bases spécifique contient au moins deux groupes appropriés pour former des liaisons hydrogène, dans une orientation, polarité et espacement compatibles avec la création d'une interaction attractive avec au moins une des bases A, T, G, C et U.

8. Méthode selon l'une quelconque des revendications 1 à 7, dans laquelle ledit composé est incapable d'interférer avec les réactions de polymérisation de nucléotides et/ou d'être incorporé dans un brin d'ADN nouvellement polymérisé.

9. Méthode selon l'une quelconque des revendications précédentes, dans laquelle l'oligonucléotide a une longueur de moins de 3 nucléotides et de préférence moins de 2 nucléotides.

10. Méthode selon l'une quelconque des revendications précédentes, dans laquelle le composé est choisi parmi l'adénosine, la guanosine, l'uridine, la cytidine, la thymidine et des mélanges de celles-ci.

11. Méthode selon l'une quelconque des revendications précédentes, dans laquelle le(s) oligonucléotide(s) ou nucléoside(s) dans le mélange d'oligonucléotides ou de nucléosides sont incapables de subir mutuellement une interaction d'appariement de bases.

12. Méthode selon la revendication 11, dans laquelle le composé inclut la cytidine et la thymidine ou la cytidine et l'adénosine ou la guanosine et la thymidine ou la guanosine et l'adénosine.

13. Méthode selon l'une quelconque des revendications 5 à 12, dans laquelle le(s) composé(s) est (sont) utilisé(s) à une concentration d'au moins 10 g/l.

14. Méthode selon l'une quelconque des revendications 1 à 13, dans laquelle le(s) composé(s) est (sont) utilisé(s) à une concentration d'au moins 25 g/l.

15. Méthode selon l'une quelconque des revendications 1 à 14, dans laquelle le(s) composé(s) porte(nt) en outre au moins un substituant.

16. Méthode selon la revendication 15, dans laquelle ledit substituant induit dans ledit composé au moins un des changements suivants :
- accroissement de la solubilité,
- changement de charge, ou
- changement de friction avec un solvant.

17. Méthode selon l'une quelconque des revendications 1 à 16, dans laquelle la mutation à détecter est une mutation ponctuelle.

18. Méthode selon l'une quelconque des revendications 1 ou 2 et 5 à 17, dans laquelle ladite mutations est détectée par un test d'hybridation.

19. Méthode selon l'une quelconque des revendications 1 à 4 ou 6 à 17, dans laquelle ladite mutation est détectée par une analyse électrophorétique en utilisant un milieu de séparation liquide.

20. Méthode selon la revendication 19, dans laquelle ledit milieu de séparation liquide contient au moins un polymère à une concentration d'au moins 1 %, et de préférence au moins 3 % en poids du poids total dudit milieu.

21. Méthode selon la revendication 19 ou 20, dans laquelle ledit milieu de séparation liquide contient au moins un polymère choisi dans le groupe constitué de polyacrylamides N,N-disubstitués et de polyacrylamides N-substitués, dans lesquels les substituants N sont choisis dans le groupe constitué d'alkyle en C₁ à C₁₂, alkyle en C₁ à C₁₂ halo-substitué, alkyle en C₁ à C₁₂ méthoxy-substitué, et alkyle en C₁ à C₁₂ hydroxyle-substitué

22. Méthode selon la revendication 20 ou 21, dans laquelle le milieu de séparation liquide contient au moins un polymère composé de plusieurs segments de polymères, ledit polymère étant de type copolymère bloc irrégulier ou de type polymère peigne irrégulier et ayant en moyenne au moins trois points de jonction établis entre des segments de polymères de différentes natures chimiques ou topologiques.

23. Méthode selon la revendication 22, dans laquelle le polymère comprend au moins un type de segment de polymère présentant, dans le milieu de séparation, une affinité spécifique pour la paroi du conduit, et au moins un type de segment de polymère présentant dans ledit milieu moins ou pas d'affinité pour la paroi.

24. Méthode selon la revendication 20 à 23, dans laquelle ledit polymère contient un acrylamide ou des acrylamides substitués.

25. Utilisation de la méthode selon l'une quelconque des revendications 1 à 24 pour diagnostiquer une prédisposition à des maladies génétiques ou des cancers associés ou putativement associés à une (des) mutation(s) ponctuelle(s) spécifique(s) ou pour le diagnostic ou le pronostic desdits maladies ou cancers.

26. Utilisation selon la revendication 25, dans laquelle ladite maladie est associée à au moins une mutation ponctuelle dans un gène humain de prédisposition au cancer mammaire (BRCA).

27. Utilisation d'une composition incluant au moins un composé capable de subir une interaction d'appariement de bases spécifique, ledit composé étant présent en une concentration d'au moins 1 g/l et étant choisi parmi un oligonucléotide ayant une longueur de moins de 5 nucléotides, un nucléoside, une base, ou un mélange de ceux-ci et, au moins un milieu de séparation liquide pour détecter la présence ou l'absence d'au moins une mutation sur un brin d'acides nucléiques appariés sous la forme d'un duplex.

28. Utilisation selon la revendication 27, dans laquelle le composé est tel que défini dans l'une quelconque des revendications 7 à 16.

29. Utilisation selon la revendication 27 ou la revendication 28, dans laquelle ledit milieu de séparation liquide est l'un de ceux définis dans les revendications 20 à 24.

30. Utilisation selon l'une quelconque des revendications 27 à 29, dans laquelle ledit milieu de séparation liquide inclut en outre au moins un composé choisi parmi :
- un polymère de tamisage
- un polymère hydrophile, et
- un polymère tensioactif.

31. Utilisation d'une composition incluant au moins un fragment d'ADN ayant une séquence nucléique apparentée à un gène sur lequel une (des) mutation(s) ponctuelle(s) a (ont) été associée(s) ou putativement associée(s) à une maladie ou une prédisposition accrue à une maladie, et au moins un composé capable de subir une interaction d'appariement de bases spécifique, le(s)dit(s) composé(s) étant utilisé(s) à une concentration combinée d'au moins 10 g/l et étant choisi(s) parmi un oligonucléotide ayant une longueur de moins de 5 nucléotides, un nucléoside, une base ou un mélange de ceux-ci pour détecter la présence ou l'absence d'au moins une mutation sur un brin d'acides nucléiques appariés sous la forme d'un duplex.

32. Utilisation d'une composition incluant au moins un composé capable de subir une interaction d'appariement de bases spécifique, ledit composé étant présent en une concentration d'au moins 1 g/l et étant choisi parmi un oligonucléotide ayant une longueur de moins de 5 nucléotides, un nucléoside, une base ou un mélange de ceux-ci, et une paire de molécules ou groupes agissant comme sonde ADN appelée « balise moléculaire » pour détecter la présence ou l'absence d'au moins une mutation sur un brin d'acides nucléiques appariés sous la forme d'un duplex.

33. Utilisation de l'une quelconque des revendications 27 à 32, pour mettre en oeuvre une méthode selon l'une quelconque des revendications 1 à 24.

34. Méthode pour détecter dans un acide nucléique une mutation comprenant au moins les étapes consistant à :
- réaliser une réaction de polymérisation en chaîne sur ledit acide nucléique en présence d'au moins deux amorces et d'un pool de composés capables de subir une interaction d'appariement de bases spécifique avec des nucléotides ou analogues de ceux-ci, lesdits composés étant présents en une concentration combinée d'au moins 10 g/l, étant choisis parmi un oligonucléotide ayant une longueur de moins de 5 nucléotides, un nucléoside, une base ou un mélange de ceux-ci et, étant incapables d'interférer avec la réaction de polymérisation en chaîne et,
- analyser et/ou quantifier les fragments d'ADN ainsi obtenus.

35. Méthode selon la revendication 33, dans laquelle le composé est tel que défini dans l'une quelconque des revendications 7 à 16.
